# EUROPEAN PATENT APPLICATION

(11) **EP 3 037 995 A2**
(43) Date of publication of application: **29.06.2016**
(21) Application number: 15001942.0
(22) Date of filing: 30.06.2015
(51) Int. Cl.: G06F 19/00

(54) **MOBILE TERMINAL AND METHOD FOR CONTROLLING THE SAME**

(30) Priority: 22.12.2014 KR 20140186256
(71) Applicant: LG Electronics Inc., Yeongdeungpo-gu Seoul 150-721 (KR)
(72) Inventor: Kim, Jungyeon, 137-893 Seoul (KR); Kim, Hyunsung, 137-893 Seoul (KR); Choi, Yongyeon, 137-893 Seoul (KR); Cho, Seongyeon, 137-893 Seoul (KR); Huh, Chanhwi, 137-893 Seoul (KR)
(74) Representative: Beyer, Andreas

(57) **Abstract**

A mobile terminal including a wireless communication configured to provide wireless communication; a display unit; and a controller configured to execute a messaging application for communicating with a virtual healthcare client, receive an input of a message to be transmitted to the virtual healthcare client, display the message transmitted to the virtual healthcare client on the display unit, extract healthcare information from the message transmitted to the virtual healthcare client, execute a healthcare application interworked with the messaging application using the extracted healthcare information, and display a reply message on the display unit including an execution result of executing the healthcare application using the extracted healthcare information.

## Description

### BACKGROUND OF THE INVENTION

### 1. Field of the Invention

The present invention relates to a mobile terminal capable of integrally providing healthcare information related to plural applications, and a method for controlling the same.

### 2. Description of the Related Art

A mobile terminal can capture still images or moving images, play music or video files, play games, receive broadcast and the like, so as to be implemented as an integrated multimedia player. Further, as applications that can execute various functions can be installed to such a mobile terminal, a user can install or delete applications corresponding to the various functions as required. Particularly, as interest in health rises these days, various applications providing services related to health may be preinstalled to the mobile terminal.

However, there has been inconvenience in that a user has to make an effort and invest time to get accustomed to such applications installed in the mobile terminal. Further, when plural applications executing the same or similar functions are installed, the same service may be provided to a user, or a user can have to input repeatedly the same information with respect to each application.

### SUMMARY OF THE INVENTION

Accordingly, one object of the present invention to provide a mobile terminal capable of inputting information to be integrally applicable to plural applications or being provided with services provided by the plural applications using a user interface (UI) familiar to a user, and a method for controlling the same.

Another object of the present invention to provide a mobile terminal capable of providing processed information by configuring applications providing the same or similar functions to actively refer to information input once, and a method for controlling the same.

To achieve these and other advantages and objects of the present invention, there is provided a mobile terminal including an analysis unit configured to analyze a transmitted message as a first application is driven in the mobile terminal; an extraction unit configured to extract healthcare information from the analyzed message, and provide the extracted information to a database connected thereto; and a controller configured to provide the transmitted message to the analysis unit, and control an operation corresponding to the analyzed message to be executed in a second application interworked with the first application, or an external terminal based on the healthcare information stored in the database, wherein the controller is configured to transmit a response message informing an execution result to the mobile terminal, and when an event satisfying a preset condition is generated, the controller generates a notification message correlating the event with the healthcare information stored in the database and transmits the notification message to the mobile terminal.

In one embodiment, the preset condition may be at least one of a time, a classification, an information providing range, a healthcare target value, an external environment condition, and wearing and taking-off the external terminal which are set through a user input, and wherein the controller is further configured to compare healthcare information provided from the first application, the second application, and the external terminal with healthcare information stored in the database to determine whether an event satisfying the preset condition has occurred.

In one embodiment, when an event attaining a preset healthcare target value occurs, the controller may be further configured to generate a notification message informing attainment of the preset healthcare target value and a next healthcare target value and transmit the notification message to the mobile terminal.

In one embodiment, when an event to wear the external terminal on a user's body is generated, the controller may be further configured to generate and transmit a first notification message informing wearing of the external terminal to the mobile terminal, and when the transmitted first notification message is confirmed, generate and transmit a second notification message informing initiation of a measurement of an amount of exercise to the mobile terminal using the worn external terminal.

In one embodiment, when the first application is installed, the controller may be further configured to set use of the first application in a message application setting mode, and when a second application to be interworked with the first application and the external terminal are registered based on a user input, allow the registered second application and external terminal to access the database.

In one embodiment, when the first application is installed, the controller may be further configured to transmit a query message inquiring basic information related to healthcare to the mobile terminal, and provide healthcare-related basic information collected based on a response to the query message, to the database.

In one embodiment, when a reply to the query message is drafted by using at least one of a key input, a voice input, a text input, an emoticon selection, and a touch gesture, the controller may be further configured to generate a next query message based on the drafted reply.

In one embodiment, the mobile terminal may further include a display unit configured to output a first screen corresponding to driving of the first application, on an execution screen of a message execution screen. And when an input is received to a view more icon displayed on the first screen is received, a second icon for inputting images related to healthcare may be displayed. And when an input applied to the second icon is received, the first screen may be converted into a screen for inputting images related to healthcare.

In one embodiment, the controller may further include a display unit configured to output a first screen corresponding to driving of the first application, and the controller may be further configured to control the transmitted message, a reply message, and a notification message to be displayed in the form of a chat box.

In one embodiment, when the analyzed message requests for sharing of a third party's healthcare information, the controller may be further configured to transmit a notification message informing the request to the third party's mobile terminal, receive the third party's healthcare information corresponding to the request based on a reply to the notification message, and display the received third party's healthcare information on the first screen in the form of a chat box.

In one embodiment, the received third party's healthcare information may have a read-only attribute.

In one embodiment, when a reply to the notification message is received, the controller may display a graphic object informing that a third party is set as a sharer, on one region of the first screen. When a sharer is selected by using the graphic object, the controller may access database in which healthcare information of the selected sharer is stored, and control the healthcare information to be provided in the form of a chat box.

In one embodiment, when a process related to the healthcare information of the selected sharer requires a personal consent, the controller may be further configured to transmit a request message asking for consent to the process, to the mobile terminal of the selected sharer, and execute a next process based on a reply to the request message.

In one embodiment, when an input applied to the more-view icon displayed on the first screen is received, a second icon requesting for sharing of the third party's healthcare information may be displayed, and when an input applied to the second icon is received, the first screen may be converted into an address directory screen for selecting the third party.

In one embodiment, when the first application is not installed in other party's mobile terminal corresponding to the request, a recommendation message inducing installation of the first application may be transmitted to the other party's mobile terminal corresponding to the request.

In one embodiment, the controller may be further configured to transmit a confirm message confirming content of the analyzed message to the mobile terminal, in response to the transmitted message, and wherein the confirm message includes at least one of a first region informing an analysis result on the transmitted plural messages in a synthesized manner, and a second region for inputting a user's confirmation or correction.

There is also provided with a method for controlling a mobile terminal, including: analyzing a transmitted message as a first application is driven in the mobile terminal; extracting healthcare information from the analyzed message, and providing the healthcare information to a database connected to an extraction unit; controlling an operation corresponding to the analyzed message to be executed in a second application interworked with the first application or in an external terminal, based on the healthcare information stored in the database; transmitting a reply message informing an execution result to the mobile terminal; and when an event satisfying a preset condition is generated, generating a notification message correlating the event with the healthcare information stored in the database, and transmitting the notification message to the mobile terminal.

In one embodiment, the preset condition may be at least one of a time, a classification, an information providing range, a healthcare target value, an external environment condition which are set through a user input, and wearing and taking-off the external terminal, and wherein generation of the event is determined based on a comparison result of the healthcare information provided from the first application, the second application and the external terminal, with the healthcare information stored in the database.

In one embodiment, the method may further include outputting a first screen as the first application is driven, and displaying the transmitted message, a reply message and a notification message on the first screen in the form of a chat box.

In one embodiment, the method may further include: when the analyzed message requests sharing of a third party's healthcare information, transmitting a notification message informing the request to the third party's mobile terminal; and displaying the third party's healthcare information corresponding to the request, on the first screen, based on a reply to the notification message in the form of a chat box.

### BRIEF DESCRIPTION OF THE DRAWINGS

The present invention will become more fully understood from the detailed description given hereinbelow and accompanying drawings which are given by illustration only, and thus are not limitative of the present invention, and wherein:
FIG. 1 is a block diagram illustrating a schematic configuration illustrating a method to integrally provide healthcare information according to an embodiment of the present invention;
FIG. 2 is a block diagram illustrating a mobile terminal to which a user interface (UI) for integrally providing healthcare information is applied according to an embodiment of the present invention;
FIGS. 3A and 3B are perspective views of the mobile terminal of FIG. 2 viewed from different directions;
FIGS. 4A through 4D are schematic views representatively illustrating a method to integrally provide healthcare information through a message-based user interface (UI) according to an embodiment of the present invention;
FIG. 5 is a flowchart illustrating a method to integrally provide healthcare information according to an embodiment of the present invention;
FIGS. 6A through 6D are schematic views illustrating methods to access a setting mode for using a method to integrally provide healthcare information according to an embodiment of the present invention;
FIGS. 7A through 7D are schematic views illustrating methods to input initial information for using a method to integrally provide healthcare information according to an embodiment of the present invention;
FIGS. 8A through 8D and 9A through 9D are schematic views illustrating methods to be provided with an alarm corresponding to a target setting in a method to integrally provide healthcare information according to an embodiment of the present invention;
FIGS. 10A through 10F are schematic views illustrating a method to integrally manage healthcare information according to an embodiment of the present invention;
FIGS. 11A through 11D and 12A through 12C are schematic views illustrating a method to share healthcare information of a third party according to an embodiment of the present invention;
FIGS. 13A and 13B are schematic views illustrating a method to draft a reply message to an query message in a method to integrally provide healthcare information according to an embodiment of the present invention; and
FIG. 14 is a flowchart illustrating a method to integrally provide healthcare information in connection with plural applications according to an embodiment of the present invention.

### DETAILED DESCRIPTION OF THE PREFERRED EMBODIMENTS

Description will now be given in detail of the embodiments, with reference to the accompanying drawings. For the sake of brief description with reference to the drawings, the same or equivalent components will be provided with the same reference numbers, and description thereof will not be repeated. A suffix "module" or "unit" used for constituent elements disclosed in the following description is merely intended for easy description of the specification, and the suffix itself does not give any special meaning or function. The accompanying drawings are used to help easily understood the technical idea of the present invention and it should be understood that the idea of the present disclosure is not limited by the accompanying drawings.

Terms such as "include" or "has" are used herein and should be understood that they are intended to indicate an existence of several components, functions or steps, disclosed in the specification, and it is also understood that greater or fewer components, functions, or steps may likewise be utilized.

A mobile terminal in the present description to which a method for providing integrally providing healthcare information, and a message-based user interface (UI) are applied may include a portable phone, a smart phone, a notebook computer, a digital broadcasting terminal, Personal Digital Assistants (PDA), Portable Multimedia Player (PMP), a navigation system, a slate PC, a tablet PC, a notebook, etc. However, the present invention may be also applicable to a fixed terminal such as a digital TV, a desktop computer and a digital signage, except for specific configurations for mobility.

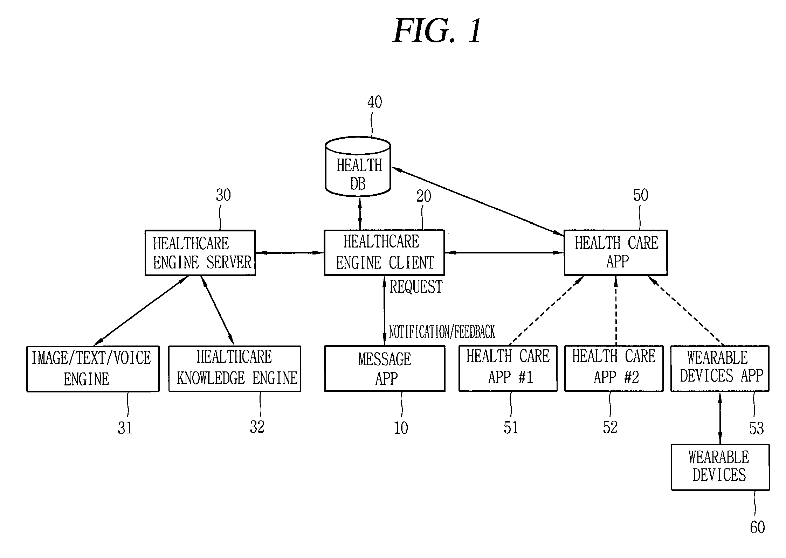

FIG. 1 is a block diagram illustrating a schematic configuration illustrating a method to integrally provide healthcare information according to an embodiment of the present invention. As shown in FIG. 1, the method for integrally providing healthcare information according to an embodiment of the present invention is implemented such that a user can input healthcare information or be provided with services from plural interworked healthcare applications by using a message-based user interface (UI) of a message application (10) driven by the mobile terminal.

Thus, the mobile terminal is provided with a healthcare application (50) for integrally providing healthcare information, and due to such a healthcare application (50), the message application (10) can access a healthcare engine client (20). Meanwhile, hereinafter, the healthcare engine client (20) may be referred to as or may be used together with a 'Healthcare Agency.'

In the present invention, a method to integrally provide healthcare information may be initiated by a step to request for desired healthcare information through a message by accessing to the healthcare engine client (20) via the message application (10). The healthcare engine client (20) provides a request message to the healthcare engine server (30) for analysis on the request message. Then, the healthcare engine server (30) analyzes a meaning of the message by using an Image/Text/Voice Engine (31) and a Healthcare Knowledge Engine (32), and transmits an analysis result to the healthcare engine client (20). If changed healthcare information exists upon the analysis result, the healthcare engine client (20) transmits the result to a health database (40) so that updated healthcare information can be stored in the healthcare database (40).

Meanwhile, the healthcare application stored in the health database (40) is actively referred to by the healthcare application (50), or other application interworked with the healthcare application (50) such as the healthcare application #1 (51), the healthcare application #2 (52), and a wearable device (53) corresponding to a wearable device (60) so that processed services can be provided. In this instance, the processed services may be provided by executing each application (51, 52 or 53) by a user, but in the present invention, it is possible to be provided with the services through the message application (10) preset to the mobile terminal in the form of a dialogue.

Further, the healthcare engine client (20) allows an operation corresponding to a request message transmitted via the message application (10) to be executed in the healthcare application (50), or other applications interworked with the healthcare application (50), for instance, the healthcare application #1 (51), the healthcare application #2 (52), or the wearable device application (53), and informs an execution result to the message application (10) in the form of a response message.

Further, when an event satisfying a preset condition is generated in the healthcare application (50), or other applications interworked with the healthcare application (50), for instance, the healthcare application #1 (51), the healthcare application #2 (52), or the wearable device application (53), the healthcare engine client (20) actively informs an alarm message related to the event through the message application (10).

Here, the 'event' satisfying a preset condition may include at least one of a specific time, a specific classification, an information providing range, a healthcare target value, and an external environment condition which are set through a user input, and a wearing or taking-off the external terminal. Such a condition may be input by a user in the form of a dialogue (or chatting) using a message application (10) according to driving of the healthcare application, and the set condition is reflected to the health database (40).

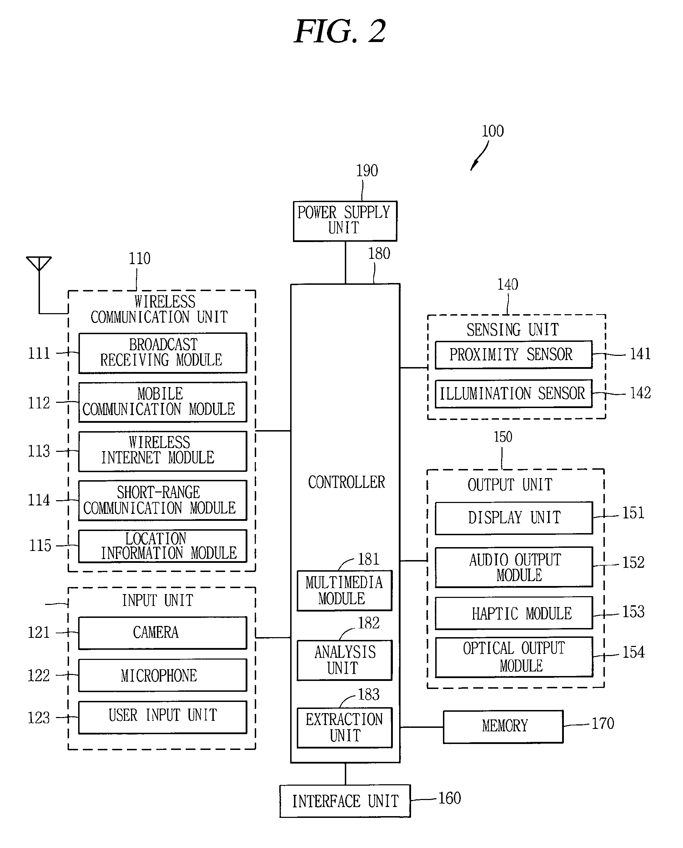

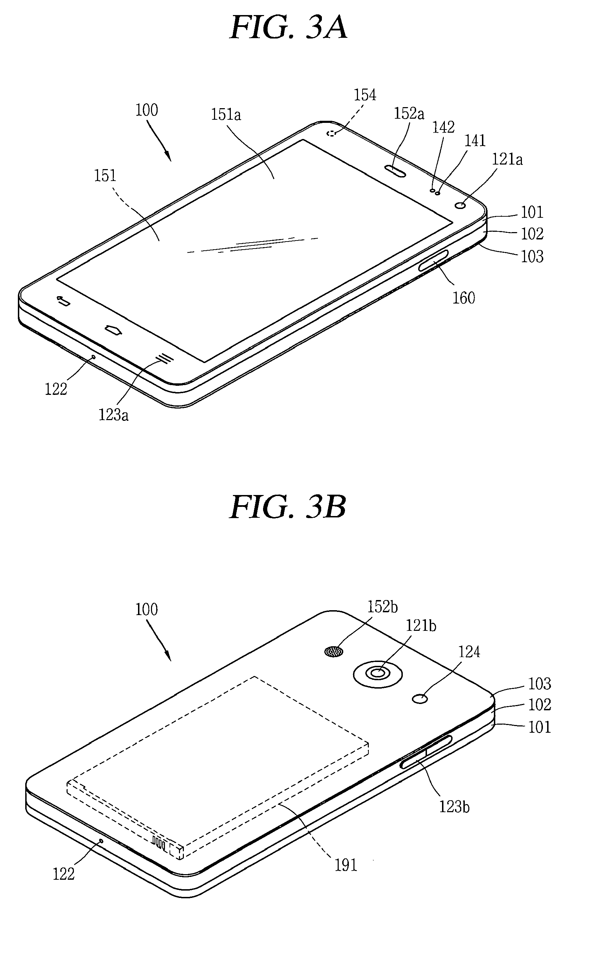

FIG. 2 is a block diagram illustrating a mobile terminal to which a user interface (UI) for integrally providing healthcare information is applied according to an embodiment of the present invention, and FIGS. 3A and 3B are perspective views of the mobile terminal of FIG. 2 viewed from different directions.

Referring to FIG. 2, the mobile terminal 100 is shown having components such as a wireless communication unit 110, an input unit 120, a sensing unit 140, an output unit 150, an interface unit 160, a memory 170, a controller 180, and a power supply unit 190. Implementing all of the illustrated components is not a requirement, and that greater or fewer components may alternatively be implemented.

The wireless communication unit 110 typically includes one or more modules which permit communications such as wireless communications between the mobile terminal 100 and a wireless communication system, communications between the mobile terminal 100 and another mobile terminal, communications between the mobile terminal 100 and an external server. Further, the wireless communication unit 110 typically includes one or more modules which connect the mobile terminal 100 to one or more networks. To facilitate such communications, the wireless communication unit 110 includes one or more of a broadcast receiving module 111, a mobile communication module 112, a wireless Internet module 113, a short-range communication module 114, and a location information module 115.

The input unit 120 includes a camera 121 for obtaining images or video, a microphone 122, which is one type of audio input device for inputting an audio signal, and a user input unit 123 (for example, a touch key, a push key, a mechanical key, a soft key, and the like) for allowing a user to input information. Data (for example, audio, video, image, and the like) is obtained by the input unit 120 and may be analyzed and processed by controller 180 according to device parameters, user commands, and combinations thereof.

The sensing unit 140 is typically implemented using one or more sensors configured to sense internal information of the mobile terminal, the surrounding environment of the mobile terminal, user information, and the like. For example, the sensing unit 140 is shown having a proximity sensor 141 and an illumination sensor 142. If desired, the sensing unit 140 may alternatively or additionally include other types of sensors or devices, such as a touch sensor, an acceleration sensor, a magnetic sensor, a G-sensor, a gyroscope sensor, a motion sensor, an RGB sensor, an infrared (IR) sensor, a finger scan sensor, a ultrasonic sensor, an optical sensor (for example, camera 121), a microphone 122, a battery gauge, an environment sensor (for example, a barometer, a hygrometer, a thermometer, a radiation detection sensor, a thermal sensor, and a gas sensor, among others), and a chemical sensor (for example, an electronic nose, a health care sensor, a biometric sensor, and the like), to name a few. The mobile terminal 100 may be configured to utilize information obtained from sensing unit 140, and in particular, information obtained from one or more sensors of the sensing unit 140, and combinations thereof.

The output unit 150 is typically configured to output various types of information, such as audio, video, tactile output, and the like. The output unit 150 is shown having a display unit 151, an audio output module 152, a haptic module 153, and an optical output module 154. The display unit 151 may have an inter-layered structure or an integrated structure with a touch sensor in order to facilitate a touch screen. The touch screen may provide an output interface between the mobile terminal 100 and a user, as well as function as the user input unit 123 which provides an input interface between the mobile terminal 100 and the user.

The interface unit 160 serves as an interface with various types of external devices that can be coupled to the mobile terminal 100. The interface unit 160, for example, may include any of wired or wireless ports, external power supply ports, wired or wireless data ports, memory card ports, ports for connecting a device having an identification module, audio input/output (I/O) ports, video I/O ports, earphone ports, and the like. In some cases, the mobile terminal 100 may perform assorted control functions associated with a connected external device, in response to the external device being connected to the interface unit 160.

The memory 170 is typically implemented to store data to support various functions or features of the mobile terminal 100. For instance, the memory 170 may be configured to store application programs executed in the mobile terminal 100, data or instructions for operations of the mobile terminal 100, and the like. Some of these application programs may be downloaded from an external server via wireless communication. Other application programs may be installed within the mobile terminal 100 at time of manufacturing or shipping, which is typically the case for basic functions of the mobile terminal 100 (for example, receiving a call, placing a call, receiving a message, sending a message, and the like). It is common for application programs to be stored in the memory 170, installed in the mobile terminal 100, and executed by the controller 180 to perform an operation (or function) for the mobile terminal 100.

The controller 180 typically functions to control overall operation of the mobile terminal 100, in addition to the operations associated with the application programs. The controller 180 can provide or process information or functions appropriate for a user by processing signals, data, information and the like, which are input or output by the various components, or activating application programs stored in the memory 170. As one example, the controller 180 controls some or all of the components so as to drive application programs that have been stored in the memory 170. For driving of the application programs, the controller 180 can operate at least two of the components included in the mobile terminal 100, through a combination.

The power supply unit 190 can be configured to receive external power or provide internal power in order to supply appropriate power required for operating elements and components included in the mobile terminal 100. The power supply unit 190 includes a battery, and the battery is configured to be built-in (mountable) or chargeable.

At least some of the components may operate in cooperation with one another in order to implement an operation, control or control method of the mobile terminal according to various embodiments to be described below. The operation, control or control method of the mobile terminal may be implemented on the mobile terminal by the execution of at least one application program stored in the memory 170.

Hereinafter, the aforementioned components will be explained in more detail with reference to FIG. 2, before various embodiments are explained. Regarding the wireless communication unit 110, the broadcast receiving module 111 is typically configured to receive a broadcast signal and/or broadcast associated information from an external broadcast managing entity via a broadcast channel. The broadcast channel may include a satellite channel, a terrestrial channel, or both. In some embodiments, two or more broadcast receiving modules 111 may be utilized to facilitate simultaneously receiving of two or more broadcast channels, or to support switching among broadcast channels.

The mobile communication module 112 can transmit and/or receive wireless signals to and from one or more network entities. Typical examples of a network entity include a base station, an external mobile terminal, a server, and the like. Such network entities form part of a mobile communication network, which is constructed according to technical standards or communication methods for mobile communications (for example, Global System for Mobile Communication (GSM), Code Division Multi Access (CDMA), CDMA2000 (Code Division Multi Access 2000), EV-DO (Enhanced Voice-Data Optimized or Enhanced Voice-Data Only), Wideband CDMA (WCDMA), High Speed Downlink Packet access (HSDPA), HSUPA (High Speed Uplink Packet Access), Long Term Evolution (LTE), LTE-A (Long Term Evolution-Advanced), and the like).

Examples of wireless signals transmitted and/or received via the mobile communication module 112 include audio call signals, video (telephony) call signals, or various formats of data to support communication of text and multimedia messages. The wireless Internet module 113 is configured to facilitate wireless Internet access. This module may be internally or externally coupled to the mobile terminal 100. The wireless Internet module 113 may transmit and/or receive wireless signals via communication networks according to wireless Internet technologies.

Examples of such wireless Internet access include Wireless LAN (WLAN), Wireless Fidelity (Wi-Fi), Wi-Fi Direct, Digital Living Network Alliance (DLNA), Wireless Broadband (WiBro), Worldwide Interoperability for Microwave Access (WiMAX), High Speed Downlink Packet Access (HSDPA), HSUPA (High Speed Uplink Packet Access), Long Term Evolution (LTE), LTE-A (Long Term Evolution-Advanced), and the like. The wireless Internet module 113 may transmit/receive data according to one or more of such wireless Internet technologies, and other Internet technologies as well.

In some embodiments, when the wireless Internet access is implemented according to, for example, WiBro, HSDPA,HSUPA, GSM, CDMA, WCDMA, LTE, LTE-A and the like, as part of a mobile communication network, the wireless Internet module 113 performs such wireless Internet access. As such, the Internet module 113 may cooperate with, or function as, the mobile communication module 112.

The short-range communication module 114 is configured to facilitate short-range communications. Suitable technologies for implementing such short-range communications include BLUETOOTH^{™}, Radio Frequency IDentification (RFID), Infrared Data Association (IrDA), Ultra-WideBand (UWB), ZigBee, Near Field Communication (NFC), Wireless-Fidelity (Wi-Fi), Wi-Fi Direct, Wireless USB (Wireless Universal Serial Bus), and the like. The short-range communication module 114 in general supports wireless communications between the mobile terminal 100 and a wireless communication system, communications between the mobile terminal 100 and another mobile terminal 100, or communications between the mobile terminal and a network where another mobile terminal 100 (or an external server) is located, via wireless area networks. One example of the wireless area networks is a wireless personal area networks.

The location information module 115 is generally configured to detect, calculate, derive or otherwise identify a position of the mobile terminal. As an example, the location information module 115 includes a Global Position System (GPS) module, a Wi-Fi module, or both. If desired, the location information module 115 may alternatively or additionally function with any of the other modules of the wireless communication unit 110 to obtain data related to the position of the mobile terminal.

As one example, when the mobile terminal uses a GPS module, a position of the mobile terminal may be acquired using a signal sent from a GPS satellite. As another example, when the mobile terminal uses the Wi-Fi module, a position of the mobile terminal can be acquired based on information related to a wireless access point (AP) which transmits or receives a wireless signal to or from the Wi-Fi module.

The input unit 120 may be configured to permit various types of input to the mobile terminal 120. Examples of such input include audio, image, video, data, and user input. Image and video input is often obtained using one or more cameras 121. Such cameras 121 may process image frames of still pictures or video obtained by image sensors in a video or image capture mode. The processed image frames can be displayed on the display unit 151 or stored in memory 170. In some cases, the cameras 121 may be arranged in a matrix configuration to permit a plurality of images having various angles or focal points to be input to the mobile terminal 100. As another example, the cameras 121 may be located in a stereoscopic arrangement to acquire left and right images for implementing a stereoscopic image.

The microphone 122 is generally implemented to permit audio input to the mobile terminal 100. The audio input can be processed in various manners according to a function being executed in the mobile terminal 100. If desired, the microphone 122 may include assorted noise removing algorithms to remove unwanted noise generated in the course of receiving the external audio.

The user input unit 123 is a component that permits input by a user. Such user input may enable the controller 180 to control operation of the mobile terminal 100. The user input unit 123 may include one or more of a mechanical input element (for example, a key, a button located on a front and/or rear surface or a side surface of the mobile terminal 100, a dome switch, a jog wheel, a jog switch, and the like), or a touch-sensitive input, among others. As one example, the touch-sensitive input may be a virtual key or a soft key, which is displayed on a touch screen through software processing, or a touch key which is located on the mobile terminal at a location that is other than the touch screen. Further, the virtual key or the visual key may be displayed on the touch screen in various shapes, for example, graphic, text, icon, video, or a combination thereof.

The sensing unit 140 is generally configured to sense one or more of internal information of the mobile terminal, surrounding environment information of the mobile terminal, user information, or the like. The controller 180 generally cooperates with the sending unit 140 to control operation of the mobile terminal 100 or execute data processing, a function or an operation associated with an application program installed in the mobile terminal based on the sensing provided by the sensing unit 140. The sensing unit 140 may be implemented using any of a variety of sensors, some of which will now be described in more detail.

The proximity sensor 141 may include a sensor to sense presence or absence of an object approaching a surface, or an object located near a surface, by using an electromagnetic field, infrared rays, or the like without a mechanical contact. The proximity sensor 141 may be arranged at an inner region of the mobile terminal covered by the touch screen, or near the touch screen.

The proximity sensor 141, for example, may include any of a transmissive type photoelectric sensor, a direct reflective type photoelectric sensor, a mirror reflective type photoelectric sensor, a high-frequency oscillation proximity sensor, a capacitance type proximity sensor, a magnetic type proximity sensor, an infrared rays proximity sensor, and the like. When the touch screen is implemented as a capacitance type, the proximity sensor 141 can sense proximity of a pointer relative to the touch screen by changes of an electromagnetic field, which is responsive to an approach of an object with conductivity. In this instance, the touch screen (touch sensor) may also be categorized as a proximity sensor.

The term "proximity touch" will often be referred to herein to denote the scenario in which a pointer is positioned to be proximate to the touch screen without contacting the touch screen. The term "contact touch" will often be referred to herein to denote the scenario in which a pointer makes physical contact with the touch screen. For the position corresponding to the proximity touch of the pointer relative to the touch screen, such position will correspond to a position where the pointer is perpendicular to the touch screen. The proximity sensor 141 may sense proximity touch, and proximity touch patterns (for example, distance, direction, speed, time, position, moving status, and the like).

In general, controller 180 processes data corresponding to proximity touches and proximity touch patterns sensed by the proximity sensor 141, and cause output of visual information on the touch screen. In addition, the controller 180 can control the mobile terminal 100 to execute different operations or process different data according to whether a touch with respect to a point on the touch screen is either a proximity touch or a contact touch.

A touch sensor can sense a touch applied to the touch screen, such as display unit 151, using any of a variety of touch methods. Examples of such touch methods include a resistive type, a capacitive type, an infrared type, and a magnetic field type, among others. As one example, the touch sensor may be configured to convert changes of pressure applied to a specific part of the display unit 151, or convert capacitance occurring at a specific part of the display unit 151, into electric input signals. The touch sensor may also be configured to sense not only a touched position and a touched area, but also touch pressure and/or touch capacitance. A touch object is generally used to apply a touch input to the touch sensor. Examples of typical touch objects include a finger, a touch pen, a stylus pen, a pointer, or the like.

When a touch input is sensed by a touch sensor, corresponding signals may be transmitted to a touch controller. The touch controller may process the received signals, and then transmit corresponding data to the controller 180. Accordingly, the controller 180 can sense which region of the display unit 151 has been touched. Here, the touch controller may be a component separate from the controller 180, the controller 180, and combinations thereof.

In some embodiments, the controller 180 can execute the same or different controls according to a type of touch object that touches the touch screen or a touch key provided in addition to the touch screen. Whether to execute the same or different control according to the object which provides a touch input may be decided based on a current operating state of the mobile terminal 100 or a currently executed application program, for example.

The touch sensor and the proximity sensor may be implemented individually, or in combination, to sense various types of touches. Such touches includes a short (or tap) touch, a long touch, a multi-touch, a drag touch, a flick touch, a pinch-in touch, a pinch-out touch, a swipe touch, a hovering touch, and the like.

If desired, an ultrasonic sensor may be implemented to recognize position information relating to a touch object using ultrasonic waves. The controller 180, for example, may calculate a position of a wave generation source based on information sensed by an illumination sensor and a plurality of ultrasonic sensors. Since light is much faster than ultrasonic waves, the time for which the light reaches the optical sensor is much shorter than the time for which the ultrasonic wave reaches the ultrasonic sensor. The position of the wave generation source may be calculated using this fact. For instance, the position of the wave generation source may be calculated using the time difference from the time that the ultrasonic wave reaches the sensor based on the light as a reference signal.

The camera 121 typically includes at least one a camera sensor (CCD, CMOS etc.), a photo sensor (or image sensors), and a laser sensor. Implementing the camera 121 with a laser sensor may allow detection of a touch of a physical object with respect to a 3D stereoscopic image. The photo sensor may be laminated on, or overlapped with, the display device. The photo sensor may be configured to scan movement of the physical object in proximity to the touch screen. In more detail, the photo sensor may include photo diodes and transistors at rows and columns to scan content received at the photo sensor using an electrical signal which changes according to the quantity of applied light. Namely, the photo sensor may calculate the coordinates of the physical object according to variation of light to thus obtain position information of the physical object.

The display unit 151 is generally configured to output information processed in the mobile terminal 100. For example, the display unit 151 may display execution screen information of an application program executing at the mobile terminal 100 or user interface (UI) and graphic user interface (GUI) information in response to the execution screen information.

In some embodiments, the display unit 151 may be implemented as a stereoscopic display unit for displaying stereoscopic images. A typical stereoscopic display unit may employ a stereoscopic display scheme such as a stereoscopic scheme (a glass scheme), an auto-stereoscopic scheme (glassless scheme), a projection scheme (holographic scheme), or the like.

The audio output module 152 is generally configured to output audio data. Such audio data may be obtained from any of a number of different sources, such that the audio data may be received from the wireless communication unit 110 or may have been stored in the memory 170. The audio data may be output during modes such as a signal reception mode, a call mode, a record mode, a voice recognition mode, a broadcast reception mode, and the like. The audio output module 152 can provide audible output related to a particular function (e.g., a call signal reception sound, a message reception sound, etc.) performed by the mobile terminal 100. The audio output module 152 may also be implemented as a receiver, a speaker, a buzzer, or the like.

A haptic module 153 can be configured to generate various tactile effects that a user feels, perceive, or otherwise experience. A typical example of a tactile effect generated by the haptic module 153 is vibration. The strength, pattern and the like of the vibration generated by the haptic module 153 can be controlled by user selection or setting by the controller. For example, the haptic module 153 may output different vibrations in a combining manner or a sequential manner.

Besides vibration, the haptic module 153 can generate various other tactile effects, including an effect by stimulation such as a pin arrangement vertically moving to contact skin, a spray force or suction force of air through a jet orifice or a suction opening, a touch to the skin, a contact of an electrode, electrostatic force, an effect by reproducing the sense of cold and warmth using an element that can absorb or generate heat, and the like. The haptic module 153 can also be implemented to allow the user to feel a tactile effect through a muscle sensation such as the user's fingers or arm, as well as transferring the tactile effect through direct contact. Two or more haptic modules 153 may be provided according to the particular configuration of the mobile terminal 100.

An optical output module 154 can output a signal for indicating an event generation using light of a light source. Examples of events generated in the mobile terminal 100 may include message reception, call signal reception, a missed call, an alarm, a schedule notice, an email reception, information reception through an application, and the like. A signal output by the optical output module 154 may be implemented so the mobile terminal emits monochromatic light or light with a plurality of colors. The signal output may be terminated as the mobile terminal senses that a user has checked the generated event, for example.

The interface unit 160 serves as an interface for external devices to be connected with the mobile terminal 100. For example, the interface unit 160 can receive data transmitted from an external device, receive power to transfer to elements and components within the mobile terminal 100, or transmit internal data of the mobile terminal 100 to such external device. The interface unit 160 may include wired or wireless headset ports, external power supply ports, wired or wireless data ports, memory card ports, ports for connecting a device having an identification module, audio input/output (I/O) ports, video I/O ports, earphone ports, or the like.

The identification module may be a chip that stores various information for authenticating authority of using the mobile terminal 100 and may include a user identity module (UIM), a subscriber identity module (SIM), a universal subscriber identity module (USIM), and the like. In addition, the device having the identification module (also referred to herein as an "identifying device") may take the form of a smart card. Accordingly, the identifying device can be connected with the terminal 100 via the interface unit 160.

When the mobile terminal 100 is connected with an external cradle, the interface unit 160 can serve as a passage to allow power from the cradle to be supplied to the mobile terminal 100 or may serve as a passage to allow various command signals input by the user from the cradle to be transferred to the mobile terminal there through. Various command signals or power input from the cradle may operate as signals for recognizing that the mobile terminal is properly mounted on the cradle.

The memory 170 can store programs to support operations of the controller 180 and store input/output data (for example, phonebook, messages, still images, videos, etc.). The memory 170 may store data related to various patterns of vibrations and audio which are output in response to touch inputs on the touch screen. The memory 170 may include one or more types of storage mediums including a Flash memory, a hard disk, a solid state disk, a silicon disk, a multimedia card micro type, a card-type memory (e.g., SD or DX memory, etc.), a Random Access Memory (RAM), a Static Random Access Memory (SRAM), a Read-Only Memory (ROM), an Electrically Erasable Programmable Read-Only Memory (EEPROM), a Programmable Read-Only memory (PROM), a magnetic memory, a magnetic disk, an optical disk, and the like. The mobile terminal 100 may also be operated in relation to a network storage device that performs the storage function of the memory 170 over a network, such as the Internet.

The controller 180 can typically control the general operations of the mobile terminal 100. For example, the controller 180 can set or release a lock state for restricting a user from inputting a control command with respect to applications when a status of the mobile terminal meets a preset condition.

Further, the controller 180 can include a multimedia module 181 for a multimedia reproduction. The multimedia module 181 may be implemented within the controller 180, but may be implemented separately from the controller 180. Further, the controller 180 can further include an analysis unit 182 for analyzing meanings of the messages transceived from the wireless communication unit 110, and an extraction unit 183 for extracting healthcare information from the analyzed message and provide the extracted healthcare information to the connected database. Here, the analysis unit 182 and the extraction unit 183 may be provided within the controller 180, as shown in FIG. 2, but may be provided separately from the controller 180. Specifically, if the analysis unit 182 and the extraction unit 183 are provided separately from the controller 180, the controller 180 can be connected to the analysis unit 182 and the extraction unit 183 only for a healthcare application for integrally providing healthcare information to the mobile terminal 100.

The controller 180 can also perform the controlling and processing associated with voice calls, data communications, video calls, and the like, or perform pattern recognition processing to recognize a handwriting input or a picture drawing input performed on the touch screen as characters or images, respectively. In addition, the controller 180 can control one or a combination of those components in order to implement various embodiments disclosed herein.

The power supply unit 190 receives external power or provide internal power and supply the appropriate power required for operating respective elements and components included in the mobile terminal 100. The power supply unit 190 may include a battery, which is typically rechargeable or be detachably coupled to the terminal body for charging.

The power supply unit 190 may include a connection port. The connection port may be configured as one example of the interface unit 160 to which an external charger for supplying power to recharge the battery is electrically connected. As another example, the power supply unit 190 may be configured to recharge the battery in a wireless manner without use of the connection port. In this example, the power supply unit 190 can receive power, transferred from an external wireless power transmitter, using at least one of an inductive coupling method which is based on magnetic induction or a magnetic resonance coupling method which is based on electromagnetic resonance.

Various embodiments described herein may be implemented in a computer-readable medium, a machine-readable medium, or similar medium using, for example, software, hardware, or any combination thereof.

Referring now to FIGS. 3A and 3B, the mobile terminal 100 is described with reference to a bar-type terminal body. However, the mobile terminal 100 may alternatively be implemented in any of a variety of different configurations. Examples of such configurations include watch-type, clip-type, glasses-type, or as a folder-type, flip-type, slide-type, swing-type, and swivel-type in which two and more bodies are combined with each other in a relatively movable manner, and combinations thereof. Discussion herein will often relate to a particular type of mobile terminal (for example, bar-type, watch-type, glasses-type, and the like). However, such teachings with regard to a particular type of mobile terminal will generally apply to other types of mobile terminals as well.

The mobile terminal 100 will generally include a case (for example, frame, housing, cover, and the like) forming the appearance of the terminal. In this embodiment, the case is formed using a front case 101 and a rear case 102. Various electronic components are incorporated into a space formed between the front case 101 and the rear case 102. At least one middle case may be additionally positioned between the front case 101 and the rear case 102.

The display unit 151 is shown located on the front side of the terminal body to output information. As illustrated, a window 151a of the display unit 151 may be mounted to the front case 101 to form the front surface of the terminal body together with the front case 101. In some embodiments, electronic components may also be mounted to the rear case 102. Examples of such electronic components include a detachable battery 191, an identification module, a memory card, and the like. Rear cover 103 is shown covering the electronic components, and this cover may be detachably coupled to the rear case 102. Therefore, when the rear cover 103 is detached from the rear case 102, the electronic components mounted to the rear case 102 are externally exposed.

As illustrated, when the rear cover 103 is coupled to the rear case 102, a side surface of the rear case 102 is partially exposed. In some cases, upon the coupling, the rear case 102 may also be completely shielded by the rear cover 103. In some embodiments, the rear cover 103 may include an opening for externally exposing a camera 121b or an audio output module 152b.

The cases 101, 102, 103 may be formed by injection-molding synthetic resin or may be formed of a metal, for example, stainless steel (STS), aluminum (Al), titanium (Ti), or the like. As an alternative to the example in which the plurality of cases form an inner space for accommodating components, the mobile terminal 100 may be configured such that one case forms the inner space. In this example, a mobile terminal 100 having a uni-body is formed so synthetic resin or metal extends from a side surface to a rear surface.

If desired, the mobile terminal 100 may include a waterproofing unit for preventing introduction of water into the terminal body. For example, the waterproofing unit may include a waterproofing member which is located between the window 151a and the front case 101, between the front case 101 and the rear case 102, or between the rear case 102 and the rear cover 103, to hermetically seal an inner space when those cases are coupled.

The display unit 151, the first audio output module 152a, the second audio output module 152b, the proximity sensor 141, the illumination sensor 142, the optical output module 154, a first camera 121a, a second camera 121b, the first manipulation unit 123a, the second manipulation unit 123b, the microphone 122, the interface 160, etc. may be provided at the mobile terminal 100.

As shown in FIGS. 3A and 3B, the display unit 151, the first audio output module 152a, the proximity sensor 141, the illumination sensor 142, the optical output module 154, the first camera 121a and the first manipulation unit 123a are arranged on a front surface of the terminal body. The second manipulation unit 123b, the microphone 122 and the interface 160 are arranged on side surfaces of the terminal body. And the second audio output module 152b and the second camera 121b are arranged on a rear surface of the terminal body.

However, alternative arrangements are possible and within the teachings of the instant disclosure. Some components may be omitted or rearranged. For example, the first manipulation unit 123a may be located on another surface of the terminal body, and the second audio output module 152b may be located on the side surface of the terminal body.

The display unit 151 outputs information processed in the mobile terminal 100. The display unit 151 may be implemented using one or more suitable mobile terminals. Examples of such suitable mobile terminals include a liquid crystal display (LCD), a thin film transistor-liquid crystal display (TFT-LCD), an organic light emitting diode (OLED), a flexible display, a 3-dimensional (3D) display, an e-ink display, and combinations thereof.

The display unit 151 may be implemented using two mobile terminals, which can implement the same or different display technology. For instance, a plurality of the display units 151 may be arranged on one side, either spaced apart from each other, or these devices may be integrated, or these devices may be arranged on different surfaces. The display unit 151 may also include a touch sensor which senses a touch input received at the display unit. When a touch is input to the display unit 151, the touch sensor may be configured to sense this touch and the controller 180, for example, may generate a control command or other signal corresponding to the touch. The content which is input in the touching manner may be a text or numerical value, or a menu item which can be indicated or designated in various modes.

The touch sensor may be configured in a form of a film having a touch pattern, disposed between the window 151a and a display on a rear surface of the window 151a, or a metal wire which is patterned directly on the rear surface of the window 151a. Alternatively, the touch sensor may be integrally formed with the display. For example, the touch sensor may be disposed on a substrate of the display or within the display. The display unit 151 may also form a touch screen together with the touch sensor. Here, the touch screen may serve as the user input unit 123 (see FIG. 1A). Therefore, the touch screen may replace at least some of the functions of the first manipulation unit 123a.

The first audio output module 152a may be implemented in the form of a receiver, and the second audio output module 152b may be implemented in the form of a loud speaker to output voice audio, alarm sounds, multimedia audio reproduction, and the like. The window 151a of the display unit 151 will typically include an aperture to permit audio generated by the first audio output module 152a to pass. One alternative is to allow audio to be released along an assembly gap between the structural bodies (for example, a gap between the window 151a and the front case 101). In this instance, a hole independently formed to output audio sounds may not be seen or is otherwise hidden in terms of appearance, thereby further simplifying the appearance and manufacturing of the mobile terminal 100.

The optical output module 154 can be configured to output light for indicating an event generation. Examples of such events include a message reception, a call signal reception, a missed call, an alarm, a schedule notice, an email reception, information reception through an application, and the like. When a user has checked a generated event, the controller can control the optical output unit 154 to stop the light output.

The first camera 121a can process image frames such as still or moving images obtained by the image sensor in a capture mode or a video call mode. The processed image frames can then be displayed on the display unit 151 or stored in the memory 170.

The first and second manipulation units 123a and 123b are examples of the user input unit 123, which may be manipulated by a user to provide input to the mobile terminal 100. The first and second manipulation units 123a and 123b may also be commonly referred to as a manipulating portion, and may employ any tactile method that allows the user to perform manipulation such as touch, push, scroll, or the like. The first and second manipulation units 123a and 123b may be implemented in a user's non-tactile manner, e.g., by a proximity touch, a hovering touch, etc.

FIG. 3A illustrates the first manipulation unit 123a as a touch key, but possible alternatives include a mechanical key, a push key, a touch key, and combinations thereof. Input received at the first and second manipulation units 123a and 123b may be used in various ways. For example, the first manipulation unit 123a may be used by the user to provide an input to a menu, home key, cancel, search, or the like, and the second manipulation unit 123b may be used by the user to provide an input to control a volume level being output from the first or second audio output modules 152a or 152b, to switch to a touch recognition mode of the display unit 151, or the like.

As another example of the user input unit 123, a rear input unit may be located on the rear surface of the terminal body. The rear input unit can be manipulated by a user to provide input to the mobile terminal 100. The input may be used in a variety of different ways. For example, the rear input unit may be used by the user to provide an input for power on/off, start, end, scroll, control volume level being output from the first or second audio output modules 152a or 152b, switch to a touch recognition mode of the display unit 151, and the like. The rear input unit may be configured to permit touch input, a push input, or combinations thereof.

The rear input unit may be located to overlap the display unit 151 of the front side in a thickness direction of the terminal body. As one example, the rear input unit may be located on an upper end portion of the rear side of the terminal body such that a user can easily manipulate it using a forefinger when the user grabs the terminal body with one hand. Alternatively, the rear input unit can be positioned at most any location of the rear side of the terminal body.

Embodiments that include the rear input unit may implement some or all of the functionality of the first manipulation unit 123a in the rear input unit. As such, in situations where the first manipulation unit 123a is omitted from the front side, the display unit 151 can have a larger screen. As a further alternative, the mobile terminal 100 may include a finger scan sensor which scans a user's fingerprint. The controller 180 can then use fingerprint information sensed by the finger scan sensor as part of an authentication procedure. The finger scan sensor may also be installed in the display unit 151 or implemented in the user input unit 123.

The microphone 122 is shown located at an end of the mobile terminal 100, but other locations are possible. If desired, multiple microphones may be implemented, with such an arrangement permitting the receiving of stereo sounds.

The interface unit 160 may serve as a path allowing the mobile terminal 100 to interface with external devices. For example, the interface unit 160 may include one or more of a connection terminal for connecting to another device (for example, an earphone, an external speaker, or the like), a port for near field communication (for example, an Infrared Data Association (IrDA) port, a Bluetooth port, a wireless LAN port, and the like), or a power supply terminal for supplying power to the mobile terminal 100. The interface unit 160 may be implemented in the form of a socket for accommodating an external card, such as Subscriber Identification Module (SIM), User Identity Module (UIM), or a memory card for information storage.

The second camera 121b is shown located at the rear side of the terminal body and includes an image capturing direction that is substantially opposite to the image capturing direction of the first camera unit 121a. If desired, second camera 121a may alternatively be located at other locations, or made to be moveable, in order to have a different image capturing direction from that which is shown.

The second camera 121b can include a plurality of lenses arranged along at least one line. The plurality of lenses may also be arranged in a matrix configuration. The cameras may be referred to as an "array camera." When the second camera 121b is implemented as an array camera, images may be captured in various manners using the plurality of lenses and images with better qualities.

A flash 124 is shown adjacent to the second camera 121b. When an image of a subject is captured with the camera 121b, the flash 124 may illuminate the subject. As shown in FIG. 3B, the second audio output module 152b can be located on the terminal body. The second audio output module 152b may implement stereophonic sound functions in conjunction with the first audio output module 152a, and may be also used for implementing a speaker phone mode for call communication.

At least one antenna for wireless communication may be located on the terminal body. The antenna may be installed in the terminal body or formed by the case. For example, an antenna which configures a part of the broadcast receiving module 111 (refer to FIG. 1A) may be retractable into the terminal body. Alternatively, an antenna may be formed using a film attached to an inner surface of the rear cover 103, or a case that includes a conductive material.

The power supply unit 190 (refer to FIG. 2) for supplying power to the mobile terminal 100 may include a battery 191, which is mounted in the terminal body or detachably coupled to an outside of the terminal body. The battery 191 may receive power via a power source cable connected to the interface unit 160. Also, the battery 191 can be recharged in a wireless manner using a wireless charger. Wireless charging may be implemented by magnetic induction or electromagnetic resonance.

The rear cover 103 is shown coupled to the rear case 102 for shielding the battery 191, to prevent separation of the battery 191, and to protect the battery 191 from an external impact or from foreign material. When the battery 191 is detachable from the terminal body, the rear case 103 may be detachably coupled to the rear case 102.

An accessory for protecting an appearance or assisting or extending the functions of the mobile terminal 100 can also be provided on the mobile terminal 100. As one example of an accessory, a cover or pouch for covering or accommodating at least one surface of the mobile terminal 100 may be provided. The cover or pouch may cooperate with the display unit 151 to extend the function of the mobile terminal 100. Another example of the accessory is a touch pen for assisting or extending a touch input to a touch screen.

As a first application for integrally providing healthcare information is driven in the mobile terminal 100 configured to include at least one of the aforementioned elements, the analysis unit 182 of the mobile terminal 100 analyzes a message transmitted through a message application of the mobile terminal 100. Then, the extraction unit 183 of the mobile terminal 100 extracts healthcare information from the message analyzed by the analysis unit 182 and provides the extracted healthcare information to a database connected to the first application.

Here, the healthcare information means all information related to a total healthcare including a medical treatment service, a prevention and management of disease for human in a broader sense, and while in a narrower sense, it means a medical measurement using a mobile terminal, a personal amount of exercise using an application, and numerical information including a blood pressure, a blood sugar, an electrocardiogram, a heartbeat, a body fat, and the like.

Meanwhile, in the present invention, the healthcare information integrally provided by the mobile terminal 100 may include not only information such as a medical measurement using a mobile terminal, a personal amount of exercise using an application, and numerical information including a blood pressure, a blood sugar, an electrocardiogram, a heartbeat and a body fat, but also data related to ubiquitous-health (U Health) that connects the mobile terminal with a hospital.

Further, the first application means a healthcare application for executing a function for integrally providing healthcare information, using a message-based user interface (UI) provided from the mobile terminal according to an embodiment of the present invention. The first application may integrally manage information or a service provided from the other healthcare application connected thereto.

The first application may be pre-installed in a manufacturing process of the mobile terminal, or installed by being downloaded according to a user's selection. Further, since whether to use the first application may be determined by a user's selection, information or service may be individually provided from each healthcare application if the first application is set not to be used, even though the first application is installed.

Further, as described above, when the analysis unit 182 and the extraction unit 183 are implemented separately from the controller 180 of the mobile terminal 100, they may execute a message analyzing function and a healthcare information extracting function only when the first application is installed and set to be used in the mobile terminal 100.

Here, the analysis unit 182 corresponds to function and the operation of the healthcare engine server 30 of FIG. 1 and the extraction unit 183 corresponds to function and operation of a healthcare engine client 20 of FIG. 1. That is, when the analysis unit 182 analyzes a meaning of a transmitted message and provides it to the extraction unit 183, the extraction unit 183 extracts healthcare information from the analyzed message and transmits it to a database through the first application.

Next, the controller 180 provides the transmitted message to the analysis unit 182, and controls a second application interworked with the first application, or an external terminal to execute an operation corresponding to the analyzed message, based on the healthcare information stored in the database.

Here, the second application or the external terminal may include a healthcare application pre-interworked with the first application or a wearable device that provides a healthcare function. For instance, the second application may include not only an exercise-related application and a menu management application, but also an application that provides an environment-related function such as a weather application. Specifically, when the second application is an exercise amount measurement application, when a message, 'let me know today's exercise amount' is transmitted, it is possible to provide information on exercise amount measured today from the exercise amount measurement application.

Here, execution of an operation corresponding to the analyzed message by the second application or the external terminal may mean that the healthcare information stored in the database is 'referred to' so that information or service provided by plural healthcare applications are provided through an integrated single message-based user interface (UI). Alternatively, execution of an operation corresponding to the second application or the analyzed message by the second application or the external terminal may mean that the healthcare information stored in the database is actively 'used' so that information or service provided by plural healthcare applications are provided through an integrated single message based user interface (UI). Further, when information or service is processed as the healthcare information stored in the database is 'referred to' or 'used', healthcare information updated based on the processed information or service is reflected to the database for reuse, re-reference, edition, and the like.

Next, the controller 180 is configured to transmit a reply message informing an execution result of an operation corresponding to the analyzed message to the mobile terminal 100. That is, when a request message requesting a user's desired healthcare information or service is transmitted to the mobile terminal 100, the mobile terminal 100 analyzes a meaning of the transmitted request message, drafts an execution result of a corresponding operation as a reply message, and informs in the form of a dialogue.

Thus, the mobile terminal 100 may use an integrated message-based user interface (UI). In this instance, a person who receives a request message or transmits a reply message is a virtual opponent, and in practice, since an information request and a reply executed in a user's mobile terminal are displayed in the form of a dialogue, data is not used even though messages are transmitted and received through the wireless communication unit 110. Thus, healthcare information can be integrally provided by using an integrated message-based user interface (UI), irrespective of when the mobile terminal 100 is connected to a Wi-Fi network, a 3G network, or a 4G network, and information on a fee system to which the mobile terminal 100 has subscribed.

Further, when an event satisfying a preset condition is generated from the mobile terminal 100, the controller 180 actively generates a notification message correlating the event with the healthcare information stored in the database, and transmits the notification message to the mobile terminal. Here, the event satisfying the preset condition may mean an event satisfying at least one of a specific time, a specific classification, an information provision range, a healthcare target value, and an external environment condition which are set through a user input, and wearing and taking-off the external terminal. That is, when it's a preset specific time, the controller 180 can transmit a notification message informing a medicine taking time to the mobile terminal. When a preset healthcare target value, for instance, target amount of exercise for one day or a target weight is attained, the controller 180 can transmit a notification message informing attainment of the target value to the mobile terminal.

As described hereinbefore, in the mobile terminal to which a user interface (UI) for integrally providing healthcare information in accordance with an embodiment of the present invention is applied, information to be integrally applied to plural healthcare applications can be input or services can be provided from the plural healthcare applications, by using a message-based interface which is familiar to a user.

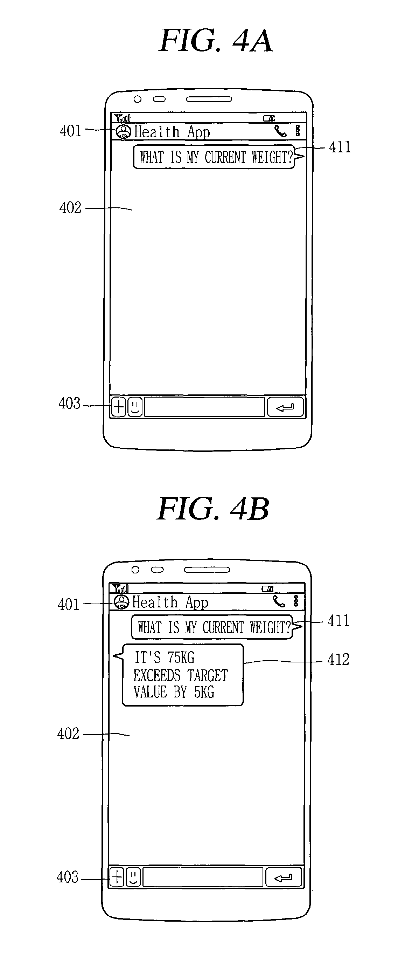

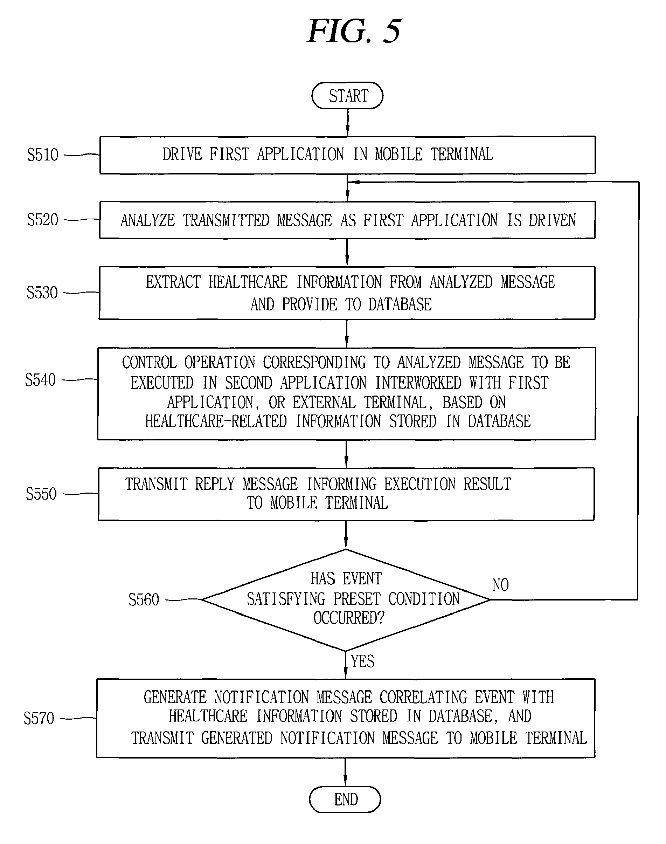

FIGS. 4A through 4D are schematic views representatively illustrating a method to integrally provide healthcare information through a message-based User interface (UI) according to an embodiment of the present invention, and FIG. 5 is a flowchart illustrating a method to integrally provide healthcare information according to an embodiment of the present invention.

Referring to FIG. 5 first, a first application is operated in the mobile terminal 100 to which a method to integrally provide healthcare information is applied and a message-based user interface therefor is applied (S510). Here, the first application means a healthcare application for integrally providing healthcare information using a message-based user interface (UI) provided by the mobile terminal according to an embodiment of the present invention. Once the first application is driven, healthcare information or a healthcare service may be provided through a message-based user interface (UI) which is familiar to a user.

As a message application is driven, a first screen corresponding to the first application may be output to the display unit 151 of the mobile terminal 100. That is, when the first application installed in the mobile terminal 100 is driven, the controller 180 can output a chat box on a screen corresponding to an execution of the first application, or a chat box screen as the existing message application is executed.

For instance, referring to FIG. 4A, a chat box region 402 and an input region 403 for inputting a message are output to a lower end of the display unit 151, and an indicator region 401 for indicating information on a chat partner is output to an upper end of the display unit 151. Through the indicator region 401, it is possible to confirm that the other party who chats with a user using a message-based user interface is a 'healthcare application (Health App).'

That is, the other party who talks to a user through a chat box or a dialog box is 'a virtual client' in accordance with execution of the first application. The 'virtual client' may correspond to the healthcare engine client 20 as aforementioned with reference to FIG. 1. The 'virtual client' may actively refer to a database in which user's healthcare information is stored, and provide processed information or service to a user in cooperation with other healthcare application or a healthcare wearable device.

Meanwhile, even when the first application is installed in the mobile terminal 100, healthcare information or service is individually provided from the other healthcare application or healthcare wearable device, when the first application is set not to be used through a user input. In this instance, it is not possible to refer to healthcare information stored in the database.

Next, as the first application is driven, a message that is transmitted through a message-based user interface of the message application of the mobile terminal is analyzed (S520). Specifically, the analysis on the transmitted message is performed by the controller 180 or by the analysis unit 182 which is separately configured from the controller 180.

In this instance, the transmitted message is displayed on the first screen of the display unit 151 in the form of a chat box. The transmitted message is displayed as if it is transmitted to the virtual client. However, healthcare information or service is not substantially provided from an external subject, but is provided within the mobile terminal (or by referring to a database connected to the mobile terminal). The analysis unit 183 of the mobile terminal 100 extracts healthcare information from the analyzed message and provides it to the database connected to the first application. The healthcare information stored in the database may be actively referred to or used by other applications or mobile terminals that have an authority to access.

Next, the controller 180 can control an operation corresponding to the analyzed message to be executed by a second application interworked with the first application or an external terminal by referring to (or using) the healthcare information stored in the database (S540). Here, the second application or the external terminal may include a healthcare application pre-interworked with the first application or a wearable device providing a healthcare function. Further, the controller 180 drafts a reply message informing an execution result of the above mentioned operation and transmits the reply message to the mobile terminal 100 (S550). Here, the transmitted reply message is displayed on the first screen of the display unit 151 in the form of a chat box. That is, the controller 180 can control a reply message received from the virtual client to be displayed on the first screen.

Referring back to FIG. 4A, when a message (411) drafted by a user using the input region (403), for instance, 'what's my current weight?' is transmitted, the analysis unit 182 analyzes a meaning of the transmitted message (411), and the extraction unit 183 may actively refer to the database in which the user's healthcare information is stored in order to figure out healthcare information extracted from the analyzed message, that is, the user's weight. For instance, when weight information stored in the database is 75 Kg, the controller 180 can be further provided with processed information or service provided from other healthcare application ('a second application') interworked with the first application and/or a healthcare wearable device ('an external terminal') based on the weight information. As a result, not only a reply message (412) as shown in FIG. 4B, for instance, 'it's 75Kg. Exceeds a target value by 5Kg,' but an additional message containing related information may be received from a healthcare application (the first application).

Meanwhile, when an event satisfying a preset condition is generated from the mobile terminal 100 (S560), the controller 180 generates a notification message by correlating the event with the healthcare information stored in the database. And, the controller 180 transmits the notification message to the mobile terminal 100 (S570). Here, the preset condition may be at least one of a specific time, a specific classification, an information provision range, a healthcare target value and an external environment condition which are set through a user input, and wearing and taking-off the external terminal. Further, the controller 180 can determine whether an event has been generated based on a comparison result obtained by comparing healthcare information provided from the first application, the second application, and the external terminal, with healthcare information stored in the database, especially healthcare information related to the preset condition.

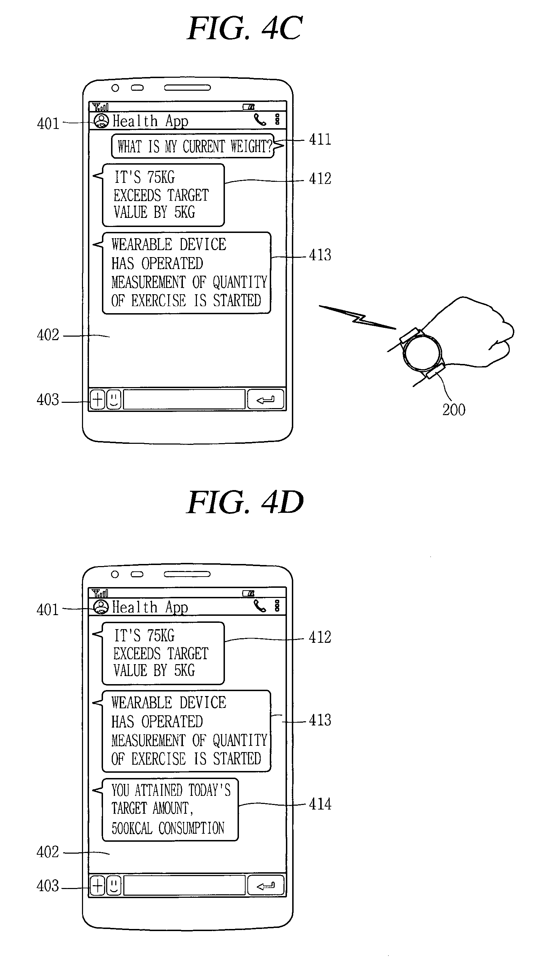

Further, the notification information is displayed on the first screen of the display unit 151 in the form of a chat box, like the reply message. That is, the controller may control the notification message received from 'the virtual client' to be displayed on the first screen. For instance, as shown in FIG. 4C, when wearing of a healthcare wearable device interworked with the first application, for instance, a watch-type terminal 200 is sensed, a notification message 413 informing wearing of the watch-type terminal 200 is received and displayed on a chat screen 402 that has a 'healthcare application' as the other party. The notification information 413 may further include not only information on wearing of the watch-type terminal 200, but also information on a healthcare function performed through the wearing of the wearable-type terminal 200, for instance, 'Wearable device has been worn. The amount of exercise will be measured.'

In this instance, when a user checks the notification message 413, for instance, when a touch input is applied to the notification message 413, the controller 180 can start measuring the amount of exercise using the watch-type terminal 200. When the measurement of the amount of exercise is terminated or the current amount of exercise reaches a preset target value of exercise, the controller 180 can display a notification message 414 shown in FIG. 4D, for instance, 'It reached today's target amount, 500kcal consumption' on the chat box region 402.

Meanwhile, when an execution screen of another application is being displayed on the display unit 151, a notification icon informing reception of a notification message is output to one region of the display unit 151. A user can access the chat box region 402 as a touch input is applied to the notification icon. As described hereinbefore, according to an embodiment of the present invention, it is possible to input healthcare information or be provided with a healthcare service by using an accustomed message-based user interface (UI) provided from a message application pre-installed to the mobile terminal. According to this, it is possible to be provided with desired healthcare information or service in the form of a dialogue, without individually executing a plurality of healthcare applications installed to the mobile terminal.

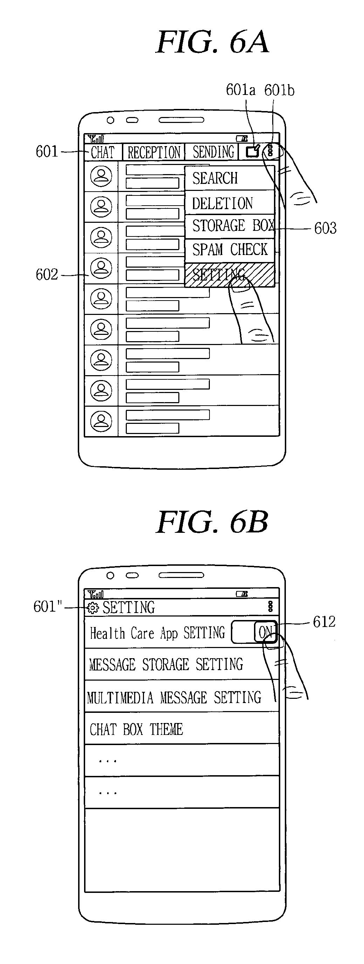

FIGS. 6A through 6D are schematic views illustrating methods to enter a setting mode for using a method to integrally provide healthcare information according to an embodiment of the present invention. When a first application is installed to the mobile terminal 100, the controller 180 can enter a setting mode of a message application and set whether to use the first application.

More specifically, when a message application is executed in the mobile terminal 100, as shown in FIG. 6A, a state region 601 for changing a state of a currently output screen may be output to an upper portion of the display unit 151, and a plurality of chat box regions 602 classified by a preset basis may be output below the state region 601. On the state region 601, displayed are an icon 601a for entering a message drafting mode and a view more icon 601b, as well as icons for changing a basis for displaying a chat box such as 'chat', 'reception,' and 'transmission.'

When an input applied to the view more icon is received, the controller 180 can popup a menu list 603. When a setting menu is selected from the popped-up menu list 603, the controller 180 can enter to a setting mode for setting whether to use the first application, as shown in FIG. 6B. In the setting mode, it is possible to select whether to use a healthcare application by changing an ON/OFF state of a switching icon 612.

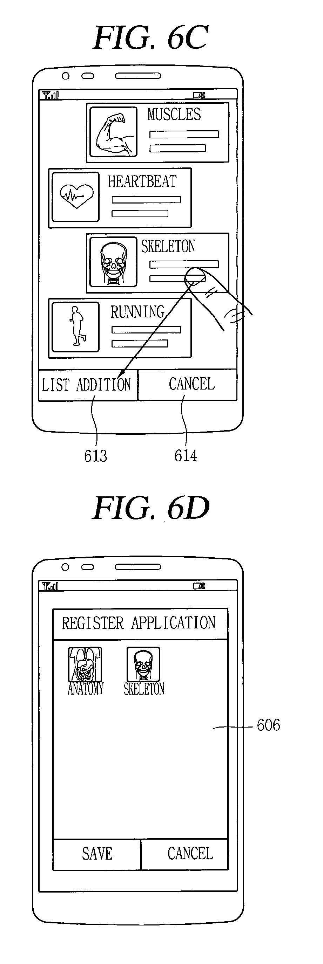

When the switching icon 612 is activated into an ON-state, the controller 180 can register a second application to be interworked with a first application and an external terminal based on a user input. For instance, as shown in FIG. 6C, as the switching icon 612 is activated into an ON-state, icons of applications pre-installed to the mobile terminal 100 and inter-workable with each other are displayed on a screen 605. A user can search an application to be interworked by inputting a search keyword or by scrolling the screen. When a touch applied to an icon of an application to be interworked with the first application is dragged to an 'addition' icon (613), the application corresponding to the touched icon is interworked with the first application. In this instance, when a user selects a 'cancel' icon (614), the interworking of the application is cancelled.

Upon completion of the selection, as shown in FIG. 6D, a list of the interworked application and the external terminal is displayed on a popup window (606). When a 'Save' icon is selected on the popup window (606), the application and the external terminal displayed on the list are finally registered as a second application to be interworked with the first application, and an external terminal. As such, once the second application to be interworked with the first application and the external terminal are registered, the controller 180 allows the registered second application and external terminal to access the database in which user's healthcare information is stored.

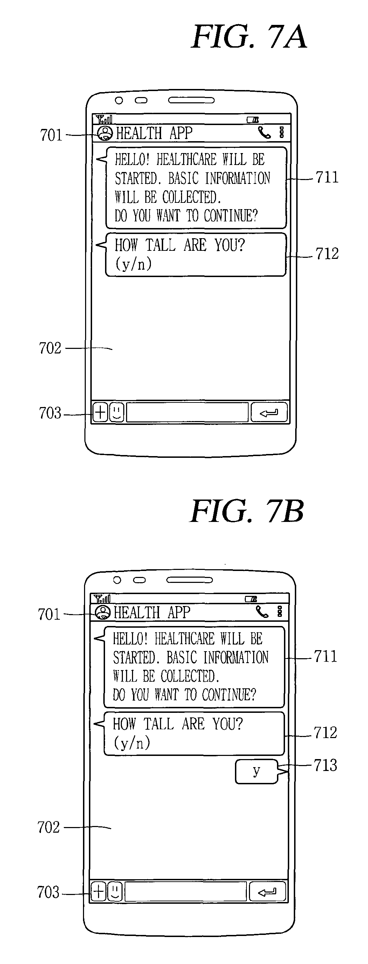

Next, FIGS. 7A through 7D are schematic views illustrating methods to input initial information for using a method to integrally provide healthcare information according to an embodiment of the present invention. When use of the first application, the second application interworked with the first application and the external terminal are registered, initial information on healthcare to be integrally used in the first application, the second application and the external terminal is input by using a message-based user interface (UI) familiar to a user.

Specifically, when the first application is installed, the controller 180 transmits an query message inquiring basic information related to healthcare to the mobile terminal, collects basic information related to healthcare based on a reply to the query message, and provides the collected basic information to the database.

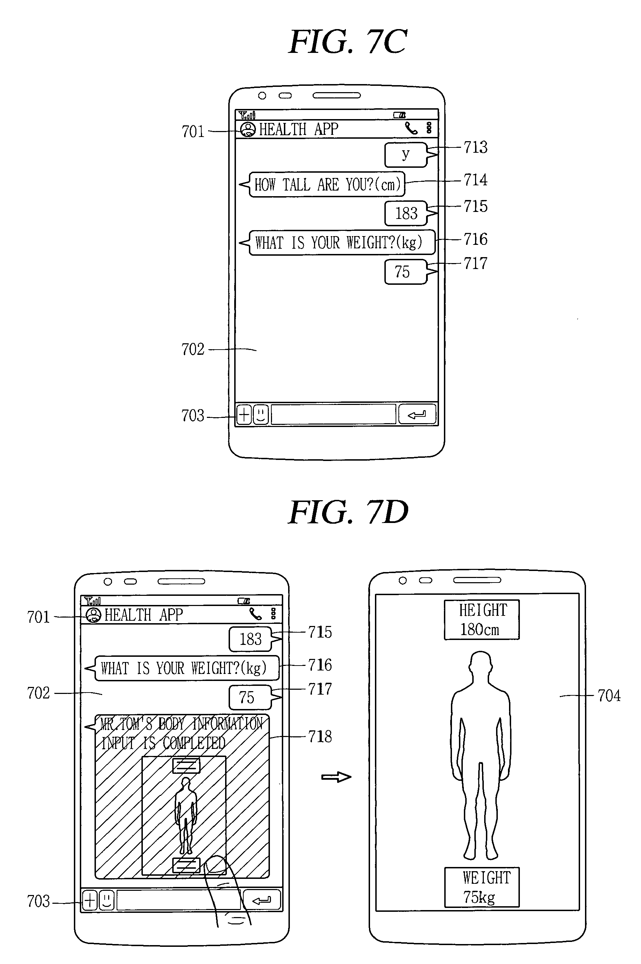

For instance, referring to FIG. 7A, as the first application is firstly driven, an intro-message (711) is displayed on a chat screen (702) which has driven a 'healthcare application' as a chat partner. Thereafter, as shown in FIG. 7B, when a user transmits a reply message (for instance, 'Y') with respect to a first query message (712) asking a consent to collection of basic information, specific query messages (714, 716) inquiring user's body information are received as shown in FIG. 7C. Here, the received query messages (714, 716) may include examples (for instance, 'example : 60') of reply messages.

In this instance, the controller 180 can draft a reply message to the query message using at least one of a key input, a voice input, a text input, an emoticon selection, and a touch gesture. In FIG. 7A, there is shown an example of a text input using an input region (703), but the present invention is not limited to that. For instance, when a user responds through a voice input as a voice recognition function and a Speech To Text (STT) function are activated in the mobile terminal 100, the input voice may be converted into a text and then displayed on a chat screen.

Further, the controller 180 can generate a next query message based on the drafted reply message. For instance, if a reply message (715) is stored in FIG. 7C, a next query message (716) is generated based on the stored response message and transmitted. If another message, for instance, 'I don't know exactly ...' is transmitted in response to the query message (714), another message, for instance, 'please let me know later' may be received instead of the query message (716).

When collection of the basic information related to healthcare is completed (or the basic information is stored) in a dialogue manner as above, the controller 180 can transmit a confirmation message confirming content of the analyzed message to the mobile terminal, in response to the transmitted message. Here, the confirmation message may include at least one of a first region that synthesizes and informs an analysis result of the transmitted plural messages, and a second region for inputting a user's confirmation or correction. For instance, as shown in FIG. 7D, an image message (718), showing at a time basic information collected in connection with healthcare from the'healthcare application', may be received. When the image message (718) is selected, the basic information collected in relation to healthcare is laid out on a related position. For instance, it can be seen that in a man's image (704) shown in FIG. 7D, information on a user's height is displayed at a head position, and information on a blood pressure is displayed at a chest position.

Meanwhile, a user can directly add or change the collected basic information anytime by transmitting a request message to the 'healthcare application' regarded as the other party. For instance, it is possible to change pre-stored weight information by transmitting a message such as 'My weight has changed to 80kg' to the'healthcare application', as a message application is executed. Further, when the changed weight information is provided from the second application interworked with the first application or the external terminal, the controller 180 can reflect such information to the database and generate a notification message informing updated weight information.

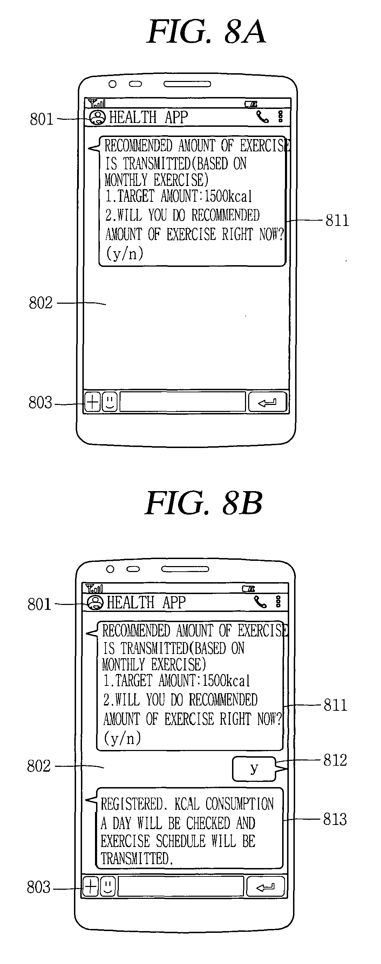

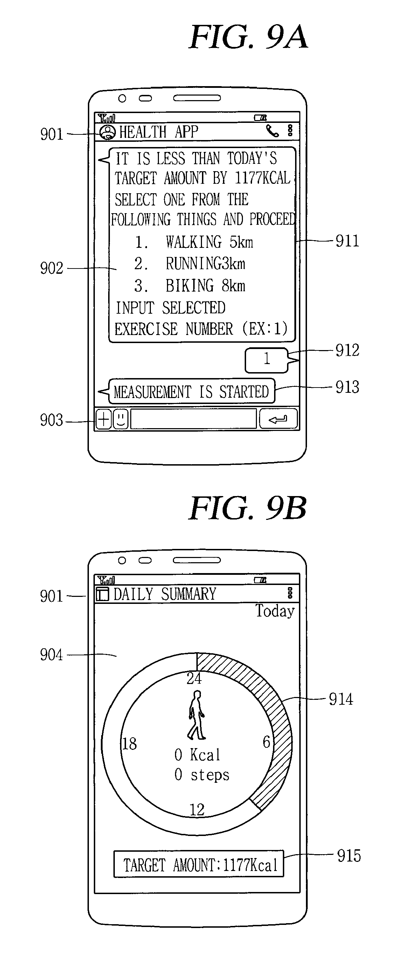

FIGS. 8A through 8D and 9A through 9D are schematic views illustrating methods to receive an alarm according to a target value setting in a method to integrally provide healthcare information according to an embodiment of the present invention. The controller 180 can set, through a user input, at least one of a specific time, a specific classification, information providing range, a healthcare target value, and an external environment condition each related to healthcare, and wearing and taking-off an external terminal related to healthcare as a condition. Here, the condition may be set through a message-based user interface (UI), and a user can reset or change anytime the preset condition by transmitting a request message to the 'healthcare application.'

Further, the controller 180 can determine whether an event satisfying the preset condition has been generated by comparing the healthcare information provided from the first application, the second application and the external terminal with the healthcare information pre-stored in the database. When an event attaining a preset healthcare target value occurs, the controller 180 can transmit a notification message informing attainment of the preset healthcare target value and a next healthcare target value to the mobile terminal 100.

For instance, when a healthcare target value is set, as shown in FIG. 8A, a notification message (811) informing a recommended amount of exercise may be received. In this instance, the notification message (811) may further include an exercise course, a recommended menu, and a recommended calorie, in addition to Body Mass Index (BMI) indicating a user's current health condition and a recommended amount of exercise corresponding to the BMI.

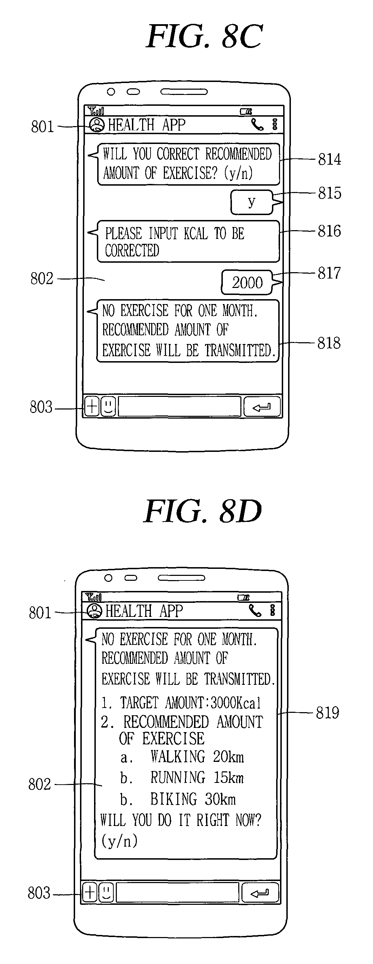

When a user transmits a reply message (812) to the received notification message (811), a notification message (813) informing that a recommended amount of exercise has been registered is received as shown in FIG. 8B, and such a history is stored in the database. The registered recommended amount of exercise may be corrected as the query messages and the reply messages (814, 815, 816, 817 and 818) are transmitted and received a predetermined number of times, as shown in FIG. 8C. In this instance, a time when the reply messages to the messages (815 and 817) transmitted by a user are received may correspond to an execution time of an operation corresponding to the transmitted messages (815 and 817). For instance, if the transmitted messages (815 and 817) are merely to be stored in the database, a next query message may be immediately received. Further, if the transmitted messages (815 and 817) include a complicated control command is completed, a next query message may be received after an operation corresponding to the control command. In this instance, a user can transmit a corrected message with respect to the pre-transmitted message until the next query message is received. For instance, a user can transmit a message for correcting or changing a preset healthcare target value, by having the 'healthcare application' as the other party or when he/she desires.

Further, when an event related to the preset healthcare target value is not generated for a preset time, that is, when a quantity of exercise is not measured or a message is not transmitted by a user for a preset time, the controller 180 can actively transmit a notification message recommending a user's exercise. For instance, as shown in FIG. 8D, it is possible to call a user's attention by retransmitting a notification message (819) recommending a user's exercise. In this instance, when the notification message recommending a user's exercise is repeated, the controller 180 can include warning words or animations for attracting a user's attention.

Further, when the preset healthcare target value is not attained, the controller 180 can continuously transmit a notification message (911) encouraging accomplishment of the target value, as shown in FIG. 9A. In this instance, the notification message (911) may include recommended courses, and when one of the proposed recommended courses is selected through a message, the controller 180 can measure a quantity of exercise through the selected course and a feedback message (913) indicating initiation of the measurement of the quantity of exercise may be received. Thus, a screen, including an image (914) visually displaying a calorie consumption amount corresponding to the selected course and numerical information (915) on remaining calorie up to the healthcare target value, may be displayed on the display unit 151.

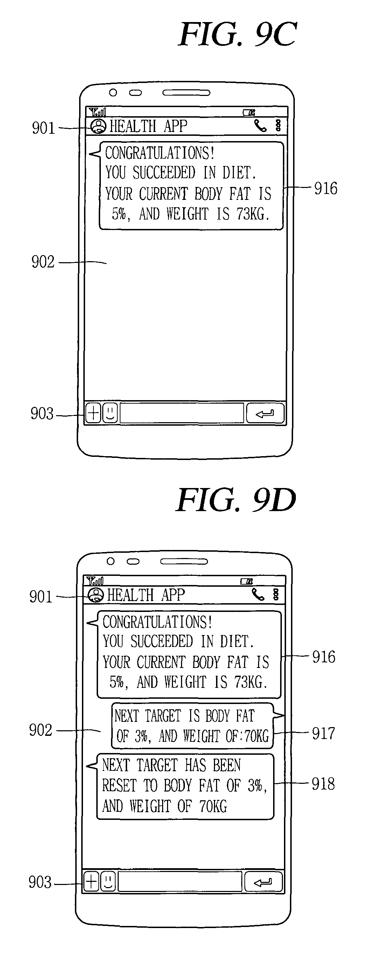

When the preset healthcare target is attained, as shown in FIG. 9C, a notification message (916), informing that the healthcare target value (for instance, 73 kg) which is set from the 'healthcare application' is attained, may be received, as shown in FIG. 9C. When a next target value is set through a message in response to the notification message (916), as shown in FIG. 9D, a confirmation message (918) confirming the next target value is received, and a reset target value may be stored in the database. Further, when a message requesting a state change based on the preset healthcare target value is transmitted, the controller 180 generates a graphic image comparing a previous state corresponding to a past input time with a current state and transmits the graphic image in the form of a message.

Further, when an event that an external terminal related to healthcare is worn on a user's body is generated, the controller 180 transmits a first notification message informing wearing of the external terminal to the mobile terminal 100. And, when the first notification message is confirmed, the controller 180 can transmit a second notification message informing that a measurement of an amount of exercise is initiated through the worn external terminal to the mobile terminal 100.

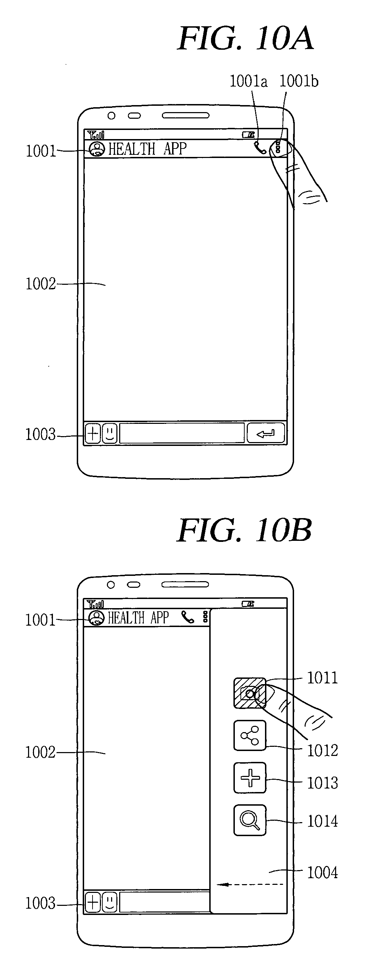

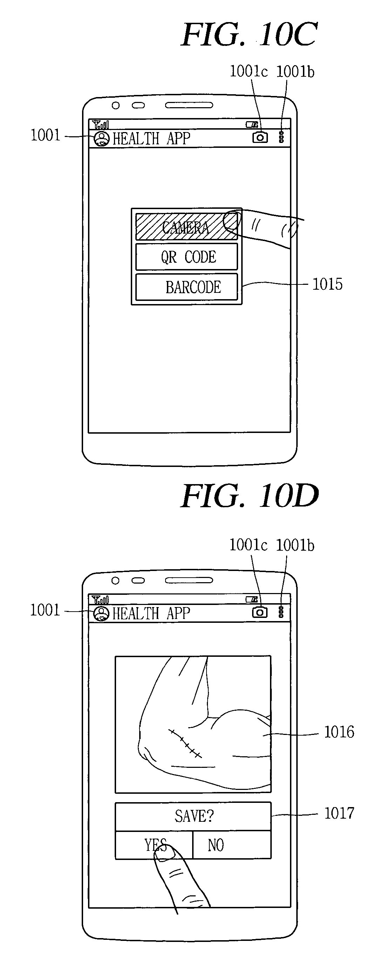

FIGS. 10A through 10F are schematic views illustrating a method to integrally manage healthcare information according to an embodiment of the present invention. As described hereinbefore, according to an embodiment of the present invention, it is possible to request or be provided with healthcare information or a healthcare service by using a message-based user interface (UI). Also, according to an embodiment of the present invention, since a user can not only request desired healthcare information or service at any time, but information or service can be provided from plural healthcare applications through an integrated means and a chat screen, it is possible to prevent loss of information and enhance a user's usability of healthcare service.

When a message application for being provided with integrated healthcare information is executed, a first screen corresponding to the first application is output to the display unit 151. Here, the first screen corresponds to a chat screen or a chat box on which a transmitted message and a received message are displayed in order of time.

On an upper end of the chat screen or chat box, as shown in FIG. 10A, a calling icon (1001a) and a view-more icon (1001b) may be displayed together with the other party's information (for instance, 'healthcare application (Health App)'). When an input applied to the more-view icon (1001b) is received, the controller 180 can output hidden icons (1011, 1012, 1013 and 1014) so that screen information (1004) may be overlapped with at least part of the chat screen (1002). When a camera icon (1011) ('a second icon') is selected from the output screen information (1004), the controller 180 can convert the chat screen (1002) into a screen for inputting an image such as a medical record.

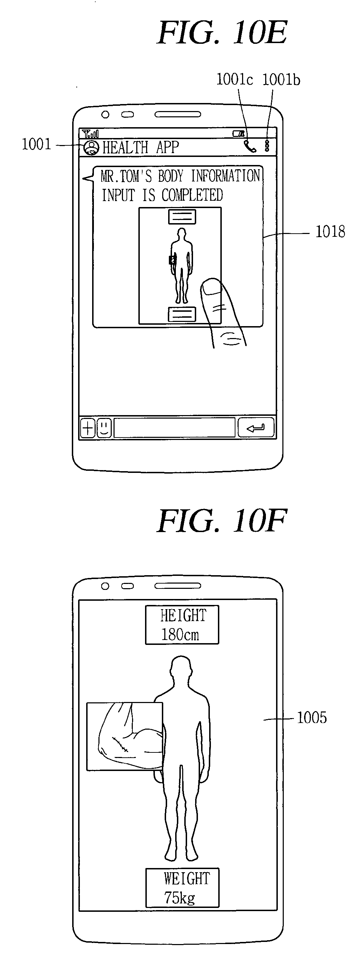

Thus, as shown in FIG. 10C, a capturing instrument item for inputting an image such as a medical record, for instance, 'a camera', 'a QR code', and 'a barcode' are displayed on a popup window (1015) on the display unit 151. For instance, when a 'camera' is selected from the popup window (1015), the camera 121 provided in the mobile terminal 100 is activated to output a preview screen. A captured mage (1016) is output to be overlapped with at least part of the chat screen (1002) as shown in FIG. 10E, and a popup message (1017) inquiring whether to store the captured image is output. When the captured image (1016) is stored, the image is generated as a standardized image (1018) so that a user can see part of the captured body at a glance, and received in the form of a message as shown in FIG. 10E. In FIG. 10E, when the received image (1018) is selected, updated healthcare information, that is, an image including part of the captured body is output on the entire display unit 151, as shown in FIG. 10F.

According to an embodiment of the present invention, medical examination records such as CT, X-Ray and In-body may be stored in the form of standardized data by actively transmitting information like a medical record in the form of a message so as to be stored in the database. Thereafter, a user can show the stored data to a doctor when seeing a doctor at a hospital, or allow the data to be accessed by a server of the hospital so that a more precise medical examination can be executed. Hereinbefore, embodiments in which a user is provided with processed information or service from plural applications using his/her healthcare information have been described.

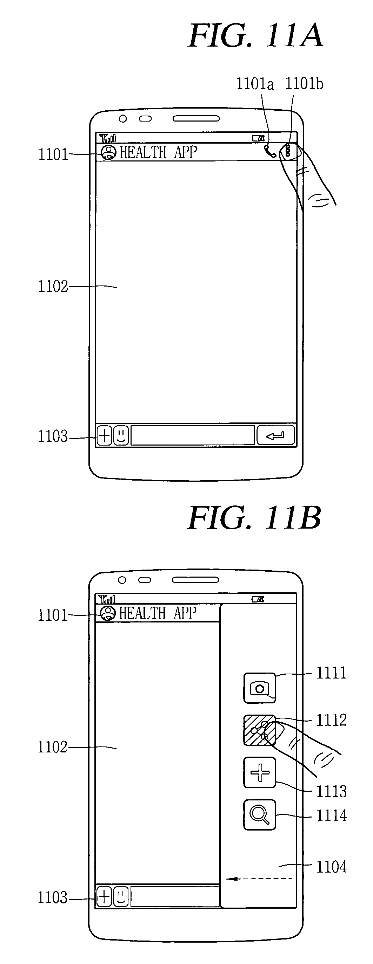

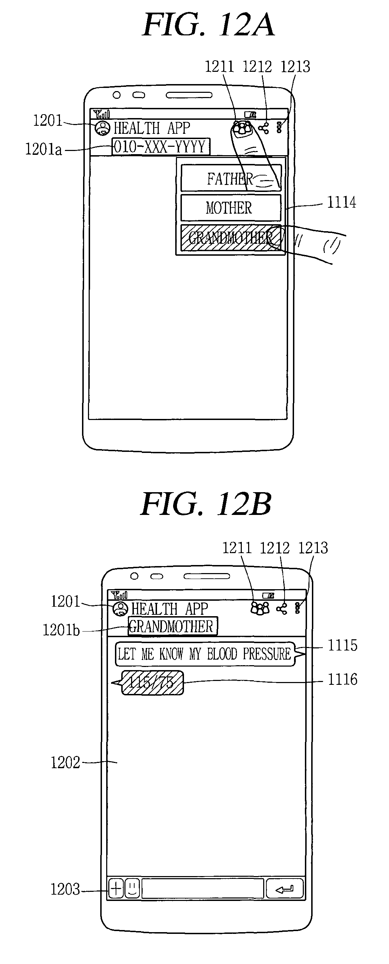

Hereinafter, a method to be integrally provided with third party's healthcare information will be described. FIGS. 11A through 11D and 12A through 12C are schematic views illustrating a method to share healthcare information of a third party using a message-based user interface (UI) according to an embodiment of the present invention. When a transmitted message is displayed on a chat screen corresponding to a first application as a message application is executed in the mobile terminal 100, the analysis unit 182 analyzes the transmitted message.

In this instance, when the analyzed message requests to share healthcare information of a third party, the controller 180 can transmit a notification message informing the request to the third party's mobile terminal. When a reply is received from the third party's mobile terminal, the controller 180 can be provided with third party's healthcare information corresponding to the request on the chat screen, based on the reply to the notification message. In this instance, a user executes communications with database in which the third party's healthcare information is stored, not with the third party's mobile terminal. That is, a user's mobile terminal 100 accesses the database in which the third party's healthcare information is stored to acquire information, and generates a reply message based on the acquired information to display on the chat screen in the form of a received message.

In this instance, the third party's healthcare information received by the mobile terminal 100 has a read-only attribute according to a user' request. This is in order to be provided with integrated healthcare information instead of the third party who is not accustomed to using a mobile terminal, and to protect the third party's right.

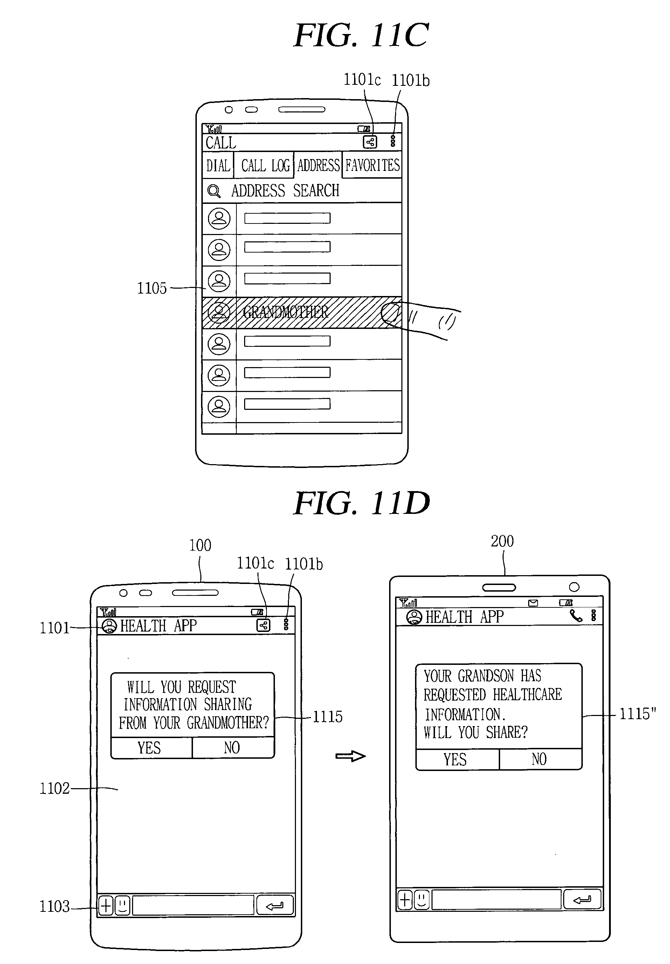

FIGS. 11A through 11D are schematic views illustrating a method to request to share healthcare information to a third party according to an embodiment of the present invention. As shown in FIG. 11A, when a more-view icon (1101b), displayed on a display region 1101 on an upper end of a chat screen 1102 which has a 'healthcare application' as the other party, is selected, hidden icons (1111, 1112, 1113 and 1114) are output in an overlapped state with at least part of the chat screen (1102), as shown in FIG. 11B. In this instance, when an icon (1112) ('a third icon') for requesting sharing of third party's healthcare information is selected, the controller 180 can convert the chat screen (1102) into an address directory screen (1105) for selecting a third party, or output the address directory screen (1105) to the chat screen (1102) in an overlapped manner, as shown in FIG. 11C.

When a third party (for instance, "grandmother') for requesting sharing of healthcare information is selected on the output address directory screen (1105), a popup window (1115) inquiring whether to transmit a message requesting sharing to the selected third party is popped up on the chat screen (1102), as shown in FIG. 11D. Here, when 'Yes' is selected, a message requesting sharing is transmitted to the selected third party's mobile terminal. As a result, a message (1115") requesting the third party's acceptance is displayed on the third party's terminal 200, as shown in FIG. 11D. When the third party has accepted the request, the third party is registered as a sharer at the user's mobile terminal 100. However, when the third party has refused, only a refusal notification message is transmitted to the user's mobile terminal 100.

When a first application for providing integrated healthcare information is not installed to the selected sharer's mobile terminal, the controller 180 can transmit a recommendation message to induce installation of the first application to the selected sharer's mobile terminal.

FIGS. 12A through 12C are schematic views illustrating a method to be provided with sharer's healthcare information by a user according to an embodiment of the present invention. As described hereinbefore, when at least one sharer is registered, as shown in FIG. 12A, a graphic object (or 'an icon') (1211), informing that a third party is set as a sharer, is displayed on a display region (1201) positioned at an upper end of a chat screen which has a 'healthcare application' as the other party. Further, information on a 'virtual client' is displayed on the display region (1201). Since a user's healthcare application server (hereinafter, 'healthcare agency') is a chat partner in FIG. 12A, user's own information, for instance, a user's mobile terminal number (1201a) may be displayed.

Meanwhile, when a touch is applied to the graphic object (1211), a list (1114) of registered sharers is popped up. When 'grandmother' is selected from the list (1114) as a sharer to be provided with healthcare information, as shown in FIG. 12A, the controller 180 can access the database in which the selected sharer's healthcare information is stored, and the chat partner is changed from 'his healthcare agency' to the 'selected sharer's healthcare agency.'

As a result, as shown in FIG. 12B, the selected sharer's information, for instance, a title (for instance, 'grandmother') (1201b) registered in the user's address directory information is displayed on the display region (1201). In this instance, the controller 180 can change at least one of a background image of the chat screen (1202), an image of the received message, and a shape of the display region (1201) so as to be visually distinguished from the previous one so that a user can easily recognize the change of the chat partner.

Thereafter, when a request message (1115) (for instance, 'let me know my blood pressure') is transmitted using an input region (1203), a reply message (1116) (for instance, '115/75') which is drafted by accessing the 'selected sharer's healthcare agency' is received. That is, the controller 180 controls the mobile terminal 100 to be provided with information in the form of a chat box by accessing the database in which the selected sharer's healthcare information is stored. In this instance, as described hereinbefore, information included in the received message (1116) has a read-only attribute, and it is required to input received information by individually executing other applications so that the user can receive more processed information.

Further, when a process related to the selected sharer's healthcare information requires one's own consent, the controller 180 can transmit a request message requiring consent of the corresponding process to the selected sharer's mobile terminal. And, the controller 180 can execute a next process based on a reply to the request message requiring consent.

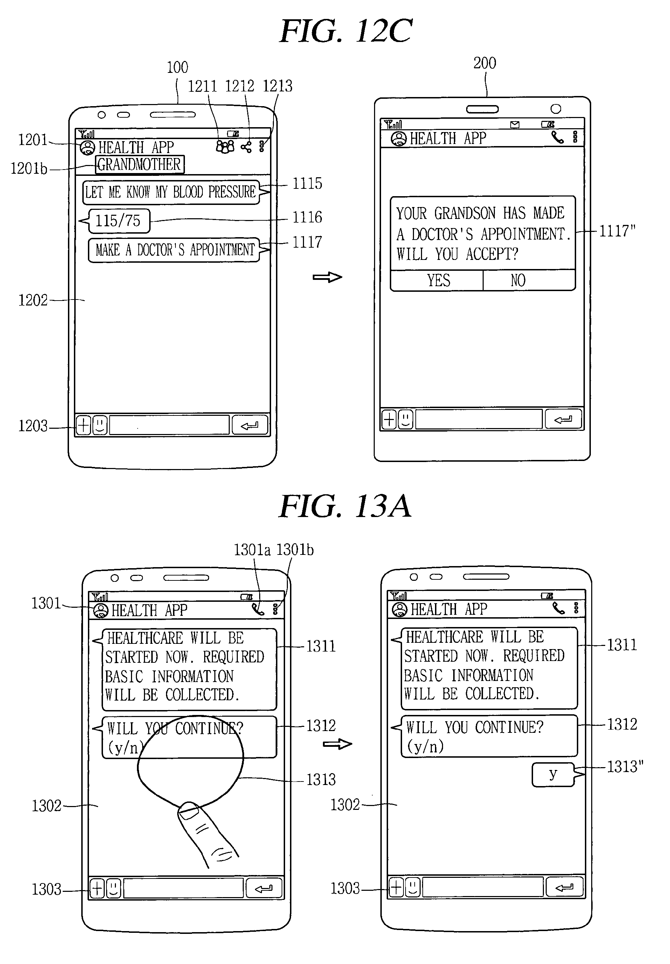

For instance, as shown in FIG. 12C, for a process requiring a sharer's consent such as 'let me have a doctor's appointment' on a chat screen (1202) which has the 'selected sharer's healthcare agency' as a chat partner, a message (1117") (for instance, 'Your grandson has made a doctor's appointment. Will you agree?') asking a consent to the process is received by the other party's mobile terminal 200.

As described hereinbefore, according to an embodiment of the present invention, it is possible to enhance a user's convenience. That is, it is possible to confirm healthcare information of a user who is not accustomed even to a message-based interface by a family member or a guardian, or to execute a related process on behalf of the user by enlarging the embodiment to provide integrated healthcare information using a message-based interface to a third party.

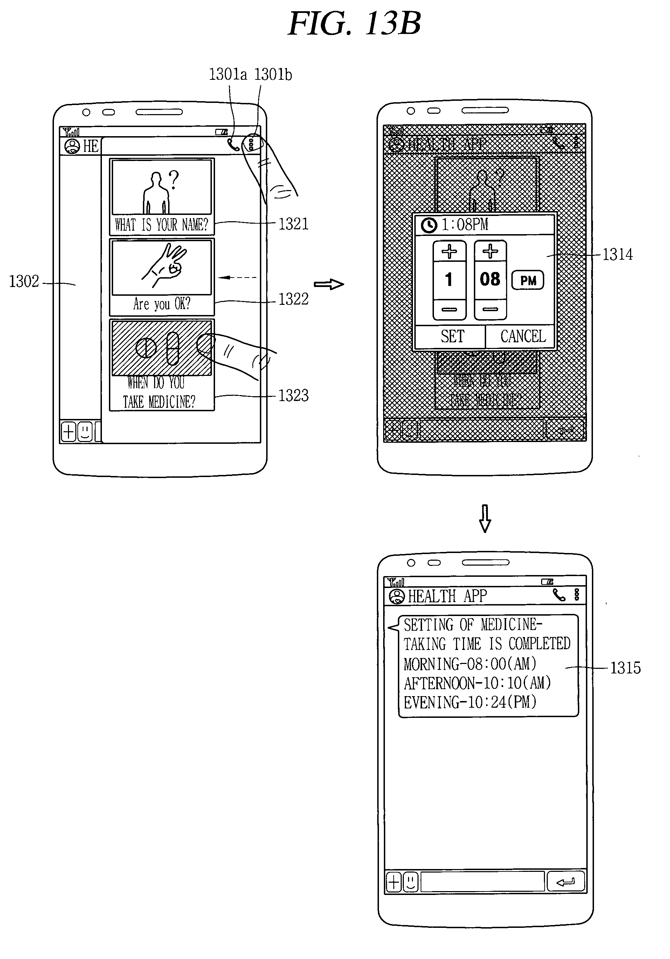

FIGS. 13A and 13B are schematic views illustrating various embodiments to draft a reply message by a user in a method to integrally provide healthcare information according to an embodiment of the present invention. At first, as shown in FIG. 13A, it is possible to apply a touch gesture to a chat screen (1302) in response to an query message (1312) (for instance, 'continue to proceed?' received on the chat screen (1302) which has 'user's own healthcare agency' as a chat partner. Specifically, when 'Y' is selected in response to the query message (1312), it is possible to draft a reply message by drawing a touchline in the form of 'O', and when 'N' is selected, by drawing a touchline in the form of `X.' The input touch gesture is converted into a text and then displayed in the form of a transmitted message (1313"), as shown in FIG. 13A.

Next, as shown in FIG. 13B, a reply message may be drafted by using various emoticons (1321, 1322 and 1323) which are displayed upon touching a view more icon (1301b) output to a display region (1301) positioned at an upper end of the chat screen (1302). For instance, when an emoticon (1323) including an image for taking medication is selected, an event requesting to set a time to take medication is automatically generated and an image (1314) corresponding to a preset scenario (for instance, 'timer setting scenario') is popped up, as shown in FIG. 13B. When a time to take medication is set according to the scenario, a confirmation message (1315) to confirm the set content is displayed as shown in FIG. 13B. Thus, it is possible to remove uncertainty due to a user's arbitrary text input or gesture input, and to draft a more correct reply message according to the pre-stored scenario.

Further, in FIG. 13A or FIG. 13B, it is also possible to draft a reply message through a text input using the input region (1303) or a key input using a key provided at one side of the mobile terminal 100. Moreover, when a chat screen which has 'user's own healthcare agency' as a chat partner is output, a voice recognition function and an STT function are automatically activated so that a reply message may be drafted through a voice input.

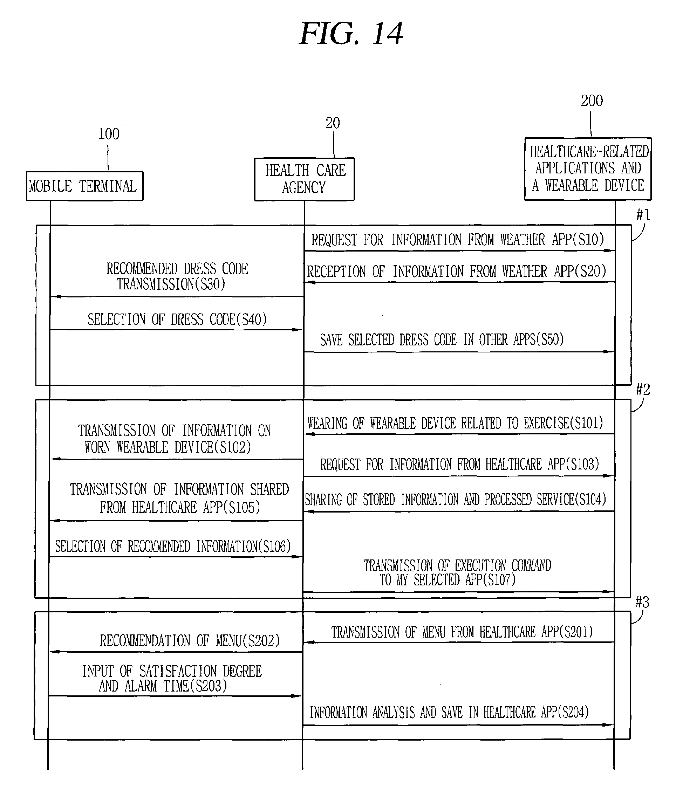

Hereinafter, referring to FIG. 14, specific examples of a method to integrally provide healthcare information by interworking with other plural applications installed to the mobile terminal 100, will be described. As shown in FIG. 14, when a first application to provide integrated healthcare information is installed and driven in the mobile terminal 100, integrated healthcare information or service may be provided in a two-way dialogue form, by having the'healthcare agency' (20) which is accessible to the database in which user's all kinds of healthcare information is stored as a chat partner. Further, the healthcare agency (20) may be communicable with other healthcare related applications associated with the first application, and a wearable device 200.

First, referring to #1 in FIG. 14, when the healthcare agency 20 requested weather information from a weather application interworked therewith (S10), weather information is received from the weather application (S20) and a processed message is generated. For instance, the healthcare agency (20) transmits a recommended dress code generated based on the received weather information to the mobile terminal 100 in the form of a message (S30). Then, the mobile terminal 100 selects a dress code and transmits a reply message to the healthcare agency (20) (S40). The healthcare agency (20) provides the selected dress code to not only the database connected thereto, but also the weather application for storage (S50). As described above, it is possible for a user to be provided with a processed service through a message-based user interface (UI) without directly executing the weather application.

Next, referring to #2 in FIG. 14, when a wearable device related to physical exercise is worn (S101), the healthcare agency (20) recognizes this and transmits information on the worn wearable device to the mobile terminal 100 in the form of a notification message (S102). Thereafter, the healthcare agency (20) requests information from the healthcare application interworked therewith (S103), and when the requested information and processed service are received (S104), the healthcare agency (20) transmits the received information and service to the mobile terminal 100 in the form of a message (S105).

When a message selecting one of recommended information is received from the mobile terminal 100 (S106), the healthcare agency (20) transmits an execution command to an application selected based on the received message (S107). As described above, a user can transmit a control command to an application related to healthcare through a message-based user interface (UI) without executing the application.

Next, referring to #3 in FIG. 14, as menu information is received from a healthcare-related application interworked with the healthcare agency (20) (S201), it is possible to transmit a menu recommendation message to the mobile terminal 100 (S202). When a message corresponding to input of a satisfaction degree and a meal alarm time are received from the mobile terminal 100 (S203), the healthcare agency (20) analyzes the received message and extracts healthcare information from the analyzed message to store it in the database. In this instance, the analyzed information may be stored in other healthcare-related applications and a wearable device 200 interworked with the healthcare agency 20, or update may be executed therein based on the analyzed information (S204). According to this, once a user inputs various healthcare information through a message-based user interface (UI), the input information is stored so as to be used in interworked other applications or wearable device.

As described hereinbefore, in the mobile terminal and the method for controlling the same in accordance with the present invention, it is possible to input information to be integrally applicable to plural healthcare applications using a message-based user interface, or to be provided with services provided from the plural healthcare applications by using a message-based user interface. Accordingly, since a user can input information or be provided with a service using an accustomed user interface provided from preinstalled message applications without spending additional efforts and time until he is accustomed to various applications, a user's convenience is enhanced. Further, it is possible to use information input once can be used in other interworked healthcare applications. Thus, there is no need to worry about providing the same or contradictory information from plural applications, and it is possible to be provided with healthcare information of a higher quality through an integrated single message means. Further, since input of information related to healthcare and request of a service are displayed on a single chat box, loss of information is prevented and search for information is facilitated so that usability can be enhanced. Moreover, in a case of a user who is not accustomed even to a message-based user interface, a third party can confirm the user's healthcare information or execute related process using the message-based interface by applying a sharing function. As a result, a user's convenience can be more enhanced.

The foregoing embodiments and advantages are merely exemplary and are not to be considered as limiting the present disclosure. The present teachings can be readily applied to other types of apparatuses. This description is intended to be illustrative, and not to limit the scope of the claims. Many alternatives, modifications, and variations will be apparent to those skilled in the art. The features, structures, methods, and other characteristics of the embodiments described herein may be combined in various ways to obtain additional and/or alternative embodiments.

As the present features may be embodied in several forms without departing from the characteristics thereof, it should also be understood that the above-described embodiments are not limited by any of the details of the foregoing description, unless Alternatively specified, but rather should be considered broadly within its scope as defined in the appended claims, and therefore all changes and modifications that fall within the metes and bounds of the claims, or equivalents of such metes and bounds are therefore intended to be embraced by the appended claims.

## Claims

1. A mobile terminal (100), comprising:
a wireless communication (110) configured to provide wireless communication;
a display unit (151); and
a controller (180) configured to:
execute a messaging application for communicating with a virtual healthcare client,
receive an input of a message to be transmitted to the virtual healthcare client,
display the message transmitted to the virtual healthcare client on the display unit,
extract healthcare information from the message transmitted to the virtual healthcare client,
execute a healthcare application interworked with the messaging application using the extracted healthcare information, and
display a reply message on the display unit including an execution result of executing the healthcare application using the extracted healthcare information.

2. The mobile terminal (100) of claim 1, wherein the controller (180) is further configured to:
display a notification message indicating additional healthcare information related to the extracted healthcare information in response to a preset condition, and
wherein the preset condition includes at least one of a time, a classification, an information providing range, a healthcare target value, an external environment condition, and putting-on or taking-off of an external terminal.

3. The mobile terminal (100) of claim 2, wherein the notification message includes information informing an attainment of a preset healthcare target value and a next healthcare target value or includes a first notification message informing the external terminal is being worn, and a second notification message informing initiation of a measurement of an amount of exercise to be performed while wearing the external terminal.

4. The mobile terminal (100) of anyone of claims 1 to 3, wherein the controller (180) is further configured to:
display a setting menu for setting use of the messaging application as a healthcare message application,
display at least one healthcare application for interworking with the messaging application, in response to the setting the use of the messaging application as the healthcare message application,
receive a selection of the at least one healthcare application, and
set the at least one health care application to interwork with the messaging application.

5. The mobile terminal (100) of claim 4, wherein the controller (180) is further configured to:
display a query message inquiring basic information related to healthcare on the display unit (151), in response to the execution of the messaging application,
receive a reply to the query message based on at least one of a key input, a voice input, a text input, an emoticon selection, and a touch gesture,
display a next query message on the display unit based on the reply, and
update a database associated with the virtual healthcare client with healthcare-related basic information collected based on a response to the query message.

6. The mobile terminal (100) of anyone of claims 1 to 5, wherein the controller (180) is further configured to:
display at least one icon on a screen of the messaging application for inputting images related to healthcare, and
convert the screen of the messaging application into a screen for inputting the images related to healthcare, in response to a selection of the displayed at least one icon.

7. The mobile terminal (100) of anyone of claims 1 to 6, wherein the controller (180) is further configured to display the communicated messages on a screen of the messaging application in a form of a chat box.

8. The mobile terminal (100) of anyone of claim 1 and claims 4 to 7, wherein the controller (180) is further configured to:
transmit a notification message informing a request to a third party's terminal for sharing of the third party's healthcare information,
receive the third party's healthcare information based on a reply to the notification message, and
display the received third party's healthcare information on a screen in the form of a chat box.

9. The mobile terminal (100) of claim 8, wherein the controller (180) is further configured to:
display a graphic object informing that the third party is set as a sharer on the display unit, in response to a reply to the notification message, and
access and control a database including healthcare information of the selected sharer to receive information in the form of a chat box, in response to a selection of the graphic object.

10. The mobile terminal (100) of claim 9, wherein the controller (180) is further configured to:
transmit a request message asking for consent of a process related to the healthcare information of the selected sharer to a terminal of the selected sharer, and
execute a next process based on a reply to the request message.

11. The mobile terminal (100) of anyone of claims 8 to 10, wherein the controller (180) is further configured to:
display an icon requesting sharing of the third party's healthcare information, and
convert the screen of the messaging application into an address directory screen for selecting the third party, in response to a selection of the icon requesting sharing of the third party's healthcare information.

12. The mobile terminal (100) of anyone of claims 8 to 11, wherein when the messaging application is not installed in the terminal (100) of the third party corresponding to the request, the controller (180) is further configured to transmit a recommendation message inducing installation of the first application to the terminal of the third party corresponding to the request.

13. The mobile terminal (100) of anyone of claims 1 to 12, wherein the virtual healthcare client is a healthcare engine client interacting and the interworked application is a database including healthcare information related to the health of a user using the mobile terminal (100).

14. A method of controlling a mobile terminal, the method comprising:
executing, via a controller of the mobile terminal, a messaging application for communicating with a virtual healthcare client;
receiving an input of a message to be transmitted to the virtual healthcare client;
displaying, via a display unit of the mobile terminal. the message transmitted to the virtual healthcare client on the display unit;
extracting, via the controller, healthcare information from the message transmitted to the virtual healthcare client;
executing, via the controller, a healthcare application interworked with the messaging application using the extracted healthcare information; and
displaying, via the display unit, a reply message on the display unit including an execution result of executing the healthcare application using the extracted healthcare information.

15. The method of claim 14, further comprising:
displaying a notification message indicating additional healthcare information related to the extracted healthcare information in response to a preset condition,
wherein the preset condition includes at least one of a time, a classification, an information providing range, a healthcare target value, an external environment condition, and putting-on or taking-off of an external terminal.
